# EUROPEAN PATENT SPECIFICATION

(11) **EP 3 994 281 B1**
(45) Date of publication and mention of the grant of the patent: **02.09.2026**
(21) Application number: 20735220.4
(22) Date of filing: 06.07.2020
(51) Int. Cl.: C12Q 1/6886

(54) **METHODS FOR DIAGNOSING THE EFFECTIVENESS OF ANTI-TUMOR TREATMENT**
VERFAHREN ZUR DIAGNOSE DER WIRKSAMKEIT EINER ANTITUMORBEHANDLUNG
PROCÉDÉS POUR LE DIAGNOSTIC DE L'EFFICACITÉ D'UN TRAITEMENT ANTITUMORAL

(30) Priority: 05.07.2019 EP 19184681
(43) Date of publication of application: 11.05.2022
(73) Proprietor: Intellexon GmbH, 82343 Pöcking (DE)
(72) Inventor: WÜRFEL, Wolfgang, 85235 Odelzhausen (DE); WIRTZ, Ralph Markus, 50935 Köln (DE); WINTERHALTER, Christoph, 82343 Pöcking (DE); WÜRFEL, Franziska, 85235 Odelzhausen (DE)
(74) Representative: Vossius & Partner Patentanwälte Rechtsanwälte mbB
(86) International application number: PCT/EP2020/068990
(87) International publication number: WO 2021/005002

(56) References cited:
- WO-A1-2017/135396
- WO-A1-2019/070755
- WO-A2-2014/162008
- US-B1- 6 790 638
- ANONYMOUS: "Abstract CT126: Soluble HLA-G and -E (sHLA-G/E) as potential biomarkers of clinical outcomes in patients (pts) with advanced, refractory squamous (SQ) NSCLC treated with nivolumab (NIVO): CheckMate 063 | Cancer Research", 1 July 2017 (2017-07-01), XP055657132, Retrieved from the Internet <URL:https://cancerres.aacrjournals.org/content/77/13_Supplement/CT126> [retrieved on 20200113]
- LAURENCE AMIOT ET AL: "Biology of HLA-G in cancer: a candidate molecule for therapeutic intervention?", CELLULAR AND MOLECULAR LIFE SCIENCES, BIRKHÄUSER-VERLAG, BA, vol. 68, no. 3, 10 November 2010 (2010-11-10), pages 417 - 431, XP019873572, ISSN: 1420-9071, DOI: 10.1007/S00018-010-0583-4
- XU HE ET AL: "HLA-G Expression in Human Breast Cancer: Implications for Diagnosis and Prognosis, and Effect on Allocytotoxic Lymphocyte Response After Hormone Treatment In Vitro", ANNALS OF SURGICAL ONCOLOGY, SPRINGER-VERLAG, NE, vol. 17, no. 5, 6 January 2010 (2010-01-06), pages 1459 - 1469, XP019785957, ISSN: 1534-4681
- DAVIDSON B ET AL: "HLA-G expression in effusions is a possible marker of tumor susceptibility to chemotherapy in ovarian carcinoma", GYNECOLOGIC ONCOLOGY, ACADEMIC PRESS, LONDON, GB, vol. 96, no. 1, 1 January 2005 (2005-01-01), pages 42 - 47, XP004676611, ISSN: 0090-8258, DOI: 10.1016/J.YGYNO.2004.09.049
- LEONE PATRIZIA ET AL: "Cancer treatment and the KIR-HLA system: an overview", CLINICAL AND EXPERIMENTAL MEDICINE, SPRINGER VERLAG, MILAN, IT, vol. 17, no. 4, 10 February 2017 (2017-02-10), pages 419 - 429, XP036345339, ISSN: 1591-8890, [retrieved on 20170210], DOI: 10.1007/S10238-017-0455-4
- M. J. RUTTEN ET AL: "HLA-G Expression Is an Independent Predictor for Improved Survival in High Grade Ovarian Carcinomas", JOURNAL OF IMMUNOLOGY RESEARCH, vol. 74, no. 5, 1 January 2014 (2014-01-01), US, pages 439 - 11, XP055227092, ISSN: 2314-8861, DOI: 10.1155/2014/274584
- N. ROUAS-FREISS: "HLA-G Proteins in Cancer: Do They Provide Tumor Cells with an Escape Mechanism?", CANCER RESEARCH, vol. 65, no. 22, 15 November 2005 (2005-11-15), pages 10139 - 10144, XP055192264, ISSN: 0008-5472, DOI: 10.1158/0008-5472.CAN-05-0097

## Description

The present invention is defined by the claims. Accordingly the present invention relates to a method for predicting whether a subject having urothelial cancer responds to an anti-PD-1 or anti-PD-L1 immune checkpoint therapy, wherein the method comprises (A) determining the level(s) of at least one nucleic acid molecule and/or at least one protein or peptide in a sample obtained from said subject, wherein the at least one nucleic acid molecule is selected from nucleic acid molecules (a) encoding a polypeptide comprising or consisting of the amino acid sequence of any one of SEQ ID NOs 2 and 4 to 6, (b) consisting of the nucleotide sequence of any one of SEQ ID NOs 8 and 10 to 12, (c) encoding a polypeptide which is at least 85% identical, preferably at least 90% identical, and most preferred at least 95% identical to the amino acid sequence of (a), (d) consisting of a nucleotide sequence which is at least 95% identical, preferably at least 96% identical, and most preferred at least 98% identical to the nucleotide sequence of (b), (e) consisting of a nucleotide sequence which is degenerate with respect to the nucleic acid molecule of (d), (f) consisting of a fragment of the nucleic acid molecule of any one of (a) to (e), said fragment comprising at least 250 nucleotides, preferably at least 300 nucleotides, more preferably at least 450 nucleotides, and most preferably at least 600 nucleotides, and (g) corresponding to the nucleic acid molecule of any one of (a) to (f), wherein T is replaced by U, and wherein the at least one protein or peptide is selected from proteins or peptides being encoded by the nucleic acid molecule of any one of (a) to (g); and (B) comparing the level(s) of (A) with the level(s) of the at least one nucleic acid molecule and/or the at least one protein or peptide in a sample obtained from one or more subjects that responded to the anti-PD-1 or anti-PD-L1 immune checkpoint therapy of (i) to (iii) or a corresponding pre-determined standard, wherein increased level(s) of (A) as compared to the level(s) or pre-determined standard of (B) indicate(s) that the subject will not respond to the anti-PD-1 or anti-PD-L1 immune checkpoint therapy and substantially the same or decreased level(s) of (A) as compared to the level(s) of (B) indicate(s) that the subject will respond to the anti-PD-1 or anti-PD-L1 immune checkpoint therapy ; or (B') comparing the level(s) of (A) with the level(s) of the at least one nucleic acid molecule and/or the at least one protein or peptide in a sample obtained from one or more subjects that did not respond to the anti-PD-1 or anti-PD-L1 immune checkpoint therapy or a corresponding pre-determined standard, wherein decreased level(s) of (A) as compared to the level(s) or pre-determined standard of (B') indicate(s) that the subject will respond to the anti-PD-1 or anti-PD-L1 immune checkpoint therapy and substantially the same or increased level(s) of (A) as compared to the level(s) of (B') indicate(s) that the subject will not respond to the tanti-PD-1 or anti-PD-L1 immune checkpoint therapy.

The human leukocyte antigen (HLA) system or complex is a gene complex encoding the major histocompatibility complex (MHC) proteins in humans. These cell-surface proteins are responsible for the regulation of the immune system in humans. The HLA gene complex resides on a 3 Mbp stretch within chromosome 6p21. Genes in this complex are categorized into three basic groups: class I, class II, and class III.

Humans have three main MHC class I genes, known as HLA-A, HLA-B, and HLA-C. The proteins produced from these genes are present on the surface of almost all cells. On the cell surface, these proteins are bound to protein fragments (peptides) that have been exported from the inside of the cell. MHC class I proteins display these peptides to the immune system. If the immune system recognizes the peptides as foreign (such as viral or bacterial peptides), it responds by triggering the infected cell to self-destruction.

There are six main MHC class II genes in humans: HLA-DPA1, HLA-DPB1, HLA-DQA1, HLA-DQB1, HLA-DRA, and HLA-DRB1. MHC class II genes provide instructions for making proteins that are present almost exclusively on the surface of certain immune system cells. Like MHC class I proteins, these proteins display peptides to the immune system.

The proteins produced from MHC class III genes have somewhat different functions; they are involved in inflammation and other immune system activities. The functions of some MHC genes are unknown.

HLA genes have many possible variations, allowing each person's immune system to react to a wide range of foreign invaders. Some HLA genes have hundreds of identified versions (alleles), each of which is given a particular number (such as HLA-B27). Closely related alleles are categorized together; for example, at least 40 very similar alleles are subtypes of HLA-B27. These subtypes are designated as HLA-B*2701 to HLA-B*2743.

More than 100 diseases have been associated with different alleles of HLA genes. For example, the HLA-B27 allele increases the risk of developing an inflammatory joint disease called ankylosing spondylitis. Many other disorders involving abnormal immune function and some forms of cancer have also been associated with specific HLA alleles. However, it is often unclear what role HLA genes play in the risk of developing these diseases.

Next to the three main MHC class I genes the non-classical MHC class I molecules HLA-E, HLA-F HLA-G are encoded by the HLA class I region. The overexpression of HLA-G, -E, and -F is a common finding across a variety of malignancies (Kochan et al., Oncoimmunology. 2013 Nov 1; 2(11): e26491.). HLA-G and HLA-E were reported as being cancer biomarkers and also as being positively correlated with poor clinical outcome of cancer.

The HLA class I region was furthermore reported to include class I pseudogenes (Hughes, Mol Biol Evol. 1995 Mar; 12(2):247-58) as well as gene fragments. For instance, HLA-H, J and L are classified as class I pseudogenes and HLA-N, S and X are classified as gene fragments. In particular, it was reported by Messer et al., J Immunol. 1992 Jun 15; 148(12):4043-53 that HLA-J is a pseudogene, due to deleterious mutations that produce a translation termination either in exon 2 or exon 4. Hence, human leukocyte antigen (HLA) genes have a long research history as important targets in biomedical science, diagnosis and treatment. WO2019/070755 discloses methods for detecting an immune checkpoint inhibitor resistance gene signature in a melanoma patient by detecting the expression or activity of a malignant cell gene signature comprising one or more genes or polypeptides selected from the group comprising HLA-H

Moreover, cancer is the second leading cause of death globally, and is responsible for an estimated 9.6 million deaths in 2018. Globally, about 1 in 6 deaths is due to cancer. The incidence of cancer is currently even increasing, *inter alia,* due to people becoming older and older. Cancer mortality can be reduced if cases are detected and treated early. In the absence of early diagnosis, patients are diagnosed at late stages when curative treatment may no longer be an option. However, even if the cancer is diagnosed at an early stage the heterogenity of tumors still often makes the finding of an efficient treatment for a particular patient difficult. This is because the bulk tumour might include a diverse collection of cells harbouring distinct molecular signatures with differential levels of sensitivity to treatment. This heterogeneity might result in a non-uniform distribution of genetically distinct tumour-cell subpopulations across and within disease sites (spatial heterogeneity) or temporal variations in the molecular makeup of cancer cells (temporal heterogeneity). Heterogeneity provides the fuel for resistance of the tumor to certain treatment options. Therefore, there is an urgent need for predicting in advance whether a subject having a tumor responds to a particular tumor therapy or not. Also there is an urgent need for new tumor therapies. These needs are addressed by the present invention.

Accordingly, the present invention relates in a first aspect to a method for predicting whether a subject having urothelial cancer responds to an anti-PD-1 or anti-PD-L1 immune checkpoint therapy, wherein the method comprises (A) determining the level(s) of at least one nucleic acid molecule and/or at least one protein or peptide in a sample obtained from said subject, wherein the at least one nucleic acid molecule is selected from nucleic acid molecules (a) encoding a polypeptide comprising or consisting of the amino acid sequence of any one of SEQ ID NOs 2 and 4 to 6, (b) consisting of the nucleotide sequence of any one of SEQ ID NOs 8 and 10 to 12, (c) encoding a polypeptide which is at least 85% identical, preferably at least 90% identical, and most preferred at least 95% identical to the amino acid sequence of (a), (d) consisting of a nucleotide sequence which is at least 95% identical, preferably at least 96% identical, and most preferred at least 98% identical to the nucleotide sequence of (b), (e) consisting of a nucleotide sequence which is degenerate with respect to the nucleic acid molecule of (d), (f) consisting of a fragment of the nucleic acid molecule of any one of (a) to (e), said fragment comprising at least 250 nucleotides, preferably at least 300 nucleotides, more preferably at least 450 nucleotides, and most preferably at least 600 nucleotides, and (g) corresponding to the nucleic acid molecule of any one of (a) to (f), wherein T is replaced by U, and wherein the at least one protein or peptide is selected from proteins or peptides being encoded by the nucleic acid molecule of any one of (a) to (g); and (B) comparing the level(s) of (A) with the level(s) of the at least one nucleic acid molecule and/or the at least one protein or peptide in a sample obtained from one or more subjects that responded to the anti-PD-1 or anti-PD-L1 immune checkpoint therapy of (i) to (iii) or a corresponding pre-determined standard, wherein increased level(s) of (A) as compared to the level(s) or pre-determined standard of (B) indicate(s) that the subject will not respond to the anti-PD-1 or anti-PD-L1 immune checkpoint therapy and substantially the same or decreased level(s) of (A) as compared to the level(s) of (B) indicate(s) that the subject will respond to the anti-PD-1 or anti-PD-L1 immune checkpoint therapy ; or (B') comparing the level(s) of (A) with the level(s) of the at least one nucleic acid molecule and/or the at least one protein or peptide in a sample obtained from one or more subjects that did not respond to the anti-PD-1 or anti-PD-L1 immune checkpoint therapy or a corresponding pre-determined standard, wherein decreased level(s) of (A) as compared to the level(s) or pre-determined standard of (B') indicate(s) that the subject will respond to the anti-PD-1 or anti-PD-L1 immune checkpoint therapy and substantially the same or increased level(s) of (A) as compared to the level(s) of (B') indicate(s) that the subject will not respond to the tanti-PD-1 or anti-PD-L1 immune checkpoint therapy.

The term "subject" in accordance with the invention refers to a mammal, preferably a domestic animal or a pet animal such as horse, cattle, pig, sheep, goat, dog or cat, and most preferably a human.

A tumor is an abnormal benign or malignant new growth of tissue that possesses no physiological function and arises from uncontrolled usually rapid cellular proliferation. The tumor is preferably cancer. Cancer is an abnormal malignant new growth of tissue that possesses no physiological function and arises from uncontrolled usually rapid cellular proliferation. The cancer is in accordance with the present invention urothelial cancer.

The nucleic acid sequences of SEQ ID NOs 7 to 12 are the genes of the human HLA genes membrane-bound HLA-G, HLA-L, soluble HLA-G, HLA-H, HLA-J, and HLA-L, respectively. In addition, the membrane bound isoforms can be released by proteolytic activity, thereby increasing the soluble fraction of HLA-G and HLA-L. It is preferred that the nucleic acid molecule according to the invention is genomic DNA or mRNA. In the case of mRNA, the nucleic acid molecule may in addition comprise a poly-A tail.

As surprisingly found in accordance with the invention and shown in the examples herein below, HLA-G is expressed as a full-length transcript and a splice form only comprising exons 1 to 5 of HLA-G. While full-length HLA-G comprises a transmembrane domain and is thus membrane-bound, soluble HLA-G lacks this transmembrane domain. It is furthermore shown in the examples that a high level of the expression of the mRNA encoding full-length HLA-G (i.e., for example, indicated by high-levels of expression measured for exons 5 and 8 or only for exon 8) as well as a high expression of the mRNA encoding the soluble form (i.e., for example, indicated by high-levels of expression measured for exons 5 and low level of exon 8, or only high level of exon 5) is associated with a tumor patient not responding to a tumor therapy as defined herein above. As depicted above, the membrane bound HLA isoforms can also be released by post-translational proteolytic cleavage to result in the release of soluble HLA fragments.

Also the gene encoding HLA-L comprises a sequence encoding a transmembrane domain. It is therefore believed that also HLA-L can be found in tumors in a full-length membrane-bound form (SEQ ID NO: 2) as well as a soluble form (SEQ ID NO: 8). Full-length HLA-L might also be released by post-translational proteolytic cleavage to result in the release of soluble HLA fragments.

On the other hand, the genes encoding HLA-H and HLA-J (SEQ ID NOs 11 and 12) do not comprise an open reading frame encoding a transmembrane domain. It is shown in the examples herein below that HLA-H and HLA-J are soluble. The examples herein below also show that a high expression of the mRNA encoding such soluble HLAs is associated with a tumor patient not responding to a tumor therapy as defined herein above.

SEQ ID NOs 1 to 6 are the amino acid sequences of human HLA genes HLA-G, HLA-L, soluble HLA-G, HLA-H, HLA-J and HLA-L protein, respectively.

The term "nucleic acid sequence" or "nucleic acid molecule" in accordance with the present invention includes DNA, such as cDNA or double or single stranded genomic DNA and RNA. In this regard, "DNA" (deoxyribonucleic acid) means any chain or sequence of the chemical building blocks adenine (A), guanine (G), cytosine (C) and thymine (T), called nucleotide bases that are linked together on a deoxyribose sugar backbone. DNA can have one strand of nucleotide bases, or two complimentary strands which may form a double helix structure. "RNA" (ribonucleic acid) means any chain or sequence of the chemical building blocks adenine (A), guanine (G), cytosine (C) and uracil (U), called nucleotide bases, that are linked together on a ribose sugar backbone. RNA typically has one strand of nucleotide bases, such as mRNA. Included are also single- and double-stranded hybrids molecules, i.e., DNA-DNA, DNA-RNA and RNA-RNA. The nucleic acid molecule may also be modified by many means known in the art. Non-limiting examples of such modifications include methylation, "caps", substitution of one or more of the naturally occurring nucleotides with an analog, and internucleotide modifications such as, for example, those with uncharged linkages (e.g., methyl phosphonates, phosphotriesters, phosphoroamidates, carbamates, etc.) and with charged linkages (e.g., phosphorothioates, phosphorodithioates, etc.). Nucleic acid molecules, in the following also referred as polynucleotides, may contain one or more additional covalently linked moieties, such as, for example, proteins (e.g., nucleases, toxins, antibodies, signal peptides, poly-L-lysine, etc.), intercalators (e.g., acridine, psoralen, etc.), chelators (e.g., metals, radioactive metals, iron, oxidative metals, etc.), and alkylators. The polynucleotides may be derivatized by formation of a methyl or ethyl phosphotriester or an alkyl phosphoramidate linkage. Further included are nucleic acid mimicking molecules known in the art such as synthetic or semi-synthetic derivatives of DNA or RNA and mixed polymers. Such nucleic acid mimicking molecules or nucleic acid derivatives according to the invention include phosphorothioate nucleic acid, phosphoramidate nucleic acid, 2'-O-methoxyethyl ribonucleic acid, morpholino nucleic acid, hexitol nucleic acid (HNA), peptide nucleic acid (PNA) and locked nucleic acid (LNA) (see Braasch and Corey, Chem Biol 2001, 8: 1). LNA is an RNA derivative in which the ribose ring is constrained by a methylene linkage between the 2'-oxygen and the 4'-carbon. Also included are nucleic acids containing modified bases, for example thio-uracil, thio-guanine and fluoro-uracil. A nucleic acid molecule typically carries genetic information, including the information used by cellular machinery to make proteins and/or polypeptides. The nucleic acid molecule may additionally comprise promoters, enhancers, response elements, signal sequences, polyadenylation sequences, introns, 5'- and 3'- non-coding regions, and the like.

The term "protein" as used herein interchangeably with the term "polypeptide" describes linear molecular chains of amino acids, including single chain proteins or their fragments, containing at least 50 amino acids. The term "peptide" as used herein describes a group of molecules consisting of up to 49 amino acids, whereas the term "polypeptide" (also referred to as "protein") as used herein describes a group of molecules consisting of at least 50 amino acids. The term "peptide" as used herein describes a group of molecules consisting with increased preference of at least 15 amino acids, at least 20 amino acids at least 25 amino acids, and at least 40 amino acids. The group of peptides and polypeptides are referred to together by using the term "(poly)peptide". (Poly)peptides may further form oligomers consisting of at least two identical or different molecules. The corresponding higher order structures of such multimers are, correspondingly, termed homo- or heterodimers, homo- or heterotrimers etc. For example, the HLA proteins comprise cysteins and thus potential dimerization sites. Furthermore, peptidomimetics of such proteins/(poly)peptides where amino acid(s) and/or peptide bond(s) have been replaced by functional analogues are also encompassed by the invention. Such functional analogues include all known amino acids other than the 20 gene-encoded amino acids, such as selenocysteine. The terms "(poly)peptide" and "protein" also refer to naturally modified (poly)peptides and proteins where the modification is effected e.g. by glycosylation, acetylation, phosphorylation and similar modifications which are well known in the art.

In accordance with the present invention, the term "percent (%) sequence identity" describes the number of matches ("hits") of identical nucleotides/amino acids of two or more aligned nucleic acid or amino acid sequences as compared to the number of nucleotides or amino acid residues making up the overall length of the template nucleic acid or amino acid sequences. In other terms, using an alignment for two or more sequences or subsequences the percentage of amino acid residues or nucleotides that are the same (e.g. 80%, 85%, 90% or 95% identity) may be determined, when the (sub)sequences are compared and aligned for maximum correspondence over a window of comparison, or over a designated region as measured using a sequence comparison algorithm as known in the art, or when manually aligned and visually inspected. This definition also applies to the complement of any sequence to be aligned.

Nucleotide and amino acid sequence analysis and alignment in connection with the present invention are preferably carried out using the NCBI BLAST algorithm (Stephen F. Altschul, Thomas L. Madden, Alejandro A. Schäffer, Jinghui Zhang, Zheng Zhang, Webb Miller, and David J. Lipman (1997), Nucleic Acids Res. 25:3389-3402). BLAST can be used for nucleotide sequences (nucleotide BLAST) and amino acid sequences (protein BLAST). The skilled person is aware of additional suitable programs to align nucleic acid sequences.

As defined herein, sequence identities of at least 85% identity, preferably at least 90% identity, and most preferred at least 95% identity are envisaged by the invention. However, also envisaged by the invention are with increasing preference sequence identities of at least 97.5%, at least 98.5%, at least 99%, at least 99.5%, at least 99.8%, and 100% identity.

The sample may be a body fluid of the subject or a tissue sample from an organ of the subject. Non-limiting examples of body fluids are whole blood, blood plasma, blood serum, urine, peritoneal fluid, and pleural fluid, liquor cerebrospinalis, tear fluid, or cells therefrom in solution. Non-limiting examples of tissue are colon, liver, breast, ovary, and testis. Tissue samples may be taken by aspiration or punctuation, excision or by any other surgical method leading to biopsy or resected cellular material. The sample may be a processed sample, e.g. a sample which has been frozen, fixed, embedded or the like. A preferred type of sample is a formaline fixed paraffin embedded (FFPE) sample. Preparation of FFPE samples is standard medical practice and these samples can be conserved for long periods of time.

Methods for obtaining the levels of the nucleic acid molecule or the protein or peptide in the context of the method the invention are established in the art.

For instance, levels of the nucleic acid molecule may be obtained by real time quantitative PCR (RT-qPCR), electrophoretic techniques or a DNA Microarray (Roth (2002), Curr. Issues Mol. Biol., 4: 93-100), wherein a RT-qPCR is preferred. In these methods the expression level may be normalized against the (mean) expression level of one or more reference genes in the sample. The term "reference gene", as used herein, is meant to refer to a gene which has a relatively invariable level of expression on the RNA transcript/mRNA level in the system which is being examined, i.e. the tumor. Such a gene may be referred to as a housekeeping gene. Non-limiting examples of reference genes are CALM2, B2M, RPL37A, GUSB, HPRT1 and GAPDH, preferably CALM2 and/or B2M. Other suitable reference genes are known to a person skilled in the art.

RT-qPCR is carried out in a thermal cycler with the capacity to illuminate each sample with a beam of light of at least one specified wavelength and detect the fluorescence emitted by the excited fluorophore. The thermal cycler is also able to rapidly heat and chill samples, thereby taking advantage of the physicochemical properties of the nucleic acids and DNA polymerase. The two common methods for the detection of PCR products in real-time qPCR are: (1) non-specific fluorescent dyes that intercalate with any double-stranded DNA, and (2) sequence-specific DNA probes consisting of oligonucleotides that are labelled with a fluorescent reporter which permits detection only after hybridization of the probe with its complementary sequence (e.g. a TaqMan probe). The probes are generally fluorescently labeled probes. Preferably, a fluorescently labeled probe consists of an oligonucleotide labeled with both a fluorescent reporter dye and a quencher dye (= dual-label probe). Suitable fluorescent reporter and quencher dyes/moieties are known to a person skilled in the art and include, but are not limited to the reporter dyes/moieties 6-FAMTM, JOETM, Cy5^{®}, Cy3^{®} and the quencher dyes/moieties dabcyl, TAMRATM, BHQTM-1, -2 or -3. Preferably primers for use in accordance with the present invention have a length of 15 to 30 nucleotides, and are in particular deoxyribonucleotides. In one embodiment, the primers are designed so as to (1) be specific for the target mRNA-sequence of as HLA gene or being derived therefrom, (2) provide an amplicon size of less than 120 bp (preferably less than 100 bp), (3) be mRNA-specific (consideration of exons/introns; preferably no amplification of genomic DNA), (4) have no tendency to dimerize and/or (5) have a melting temperature Tₘ in the range of from 58°C to 62°C (preferably, Tₘ is approximately 60°C). As mentioned, the probe is required for a RT-qPCR according to (2) but the probe can be replaced by an intercalating dye in the case of a RT-qPCR according to (1), such as SYBR green.

As one alternative of qPCR also electrophoretic techniques or as one further alternative a DNA microarray may be used to obtaining the levels of the nucleic acid molecule of the first aspect of the invention. The conventional approach to mRNA identification and quantitation is through a combination of gel electrophoresis, which provides information on size, and sequence-specific probing. The Northern blot is the most commonly applied technique in this latter class. The ribonuclease protection assay (RPA) was developed as a more sensitive, less labor-intensive alternative to the Northern blot. Hybridization is performed with a labeled ribonucleotide probe in solution, after which non-hybridized sample and probe are digested with a mixture of ribonucleases (e.g., RNase A and RNase T1) that selectively degrade single-stranded RNAs. Subsequent denaturing polyacrylamide gel electrophoresis provides a means for quantitation and also gives the size of the region hybridized by the probe. For both Northern blot and RPA, the accuracy and precision of quantitation are functions of the detection method and the reference or standard utilized. Most commonly, the probes are radiolabeled with 32P or 33P, in which case the final gel is exposed to X-ray film or phosphor screen and the intensity of each band quantified with a densitometer or phosphor imager, respectively. In both cases, the exposure time can be adjusted to suit the sensitivity required, but the phosphor-based technique is generally more sensitive and has a greater dynamic range. As an alternative to using radioactivity, probes can be labeled with an antigen or hapten, which is subsequently bound by a horseradish peroxidase- or alkaline phosphatase-conjugated antibody and quantified after addition of substrate by chemiluminescence on film or a fluorescence imager. In all of these imaging applications, subtraction of the background from a neighboring region of the gel without probe should be performed. The great advantage of the gel format is that any reference standards can be imaged simultaneously with the sample. Likewise, detection of a housekeeping gene is performed under the same conditions for all samples.

In addition, next generation sequencing (NGS) may be used (Behjati and Tarpey, Arch Dis Child Educ Pract Ed. 2013 Dec; 98(6): 236). NGS is a RNA or DNA sequencing technology which has revolutionised genomic research. Using NGS an entire human genome can be sequenced within a single day. In contrast, the previous Sanger sequencing technology, used to decipher the human genome, required over a decade to deliver the final draft. In view of the present invention NGS could be used to quantify in open configuration (genome wide exome sequencing) or as focused panel harbouring the respective HLA genes and isoforms disclosed in this application.

For the construction of DNA microarrays two technologies have emerged. Generally, the starting point in each case for the design of an array is a set of sequences corresponding to the genes or putative genes to be probed. In the first approach, oligonucleotide probes are synthesized chemically on a glass substrate. Because of the variable efficiency of oligonucleotide hybridization to cDNA probes, multiple oligonucleotide probes are synthesized complementary to each gene of interest. Furthermore, for each fully complementary oligonucleotide on the array, an oligonucleotide with a mismatch at a single nucleotide position is constructed and used for normalization. Oligonucleotide arrays are routinely created with densities of about 10⁴-10⁶ probes/cm². The second major technology for DNA microarray construction is the robotic printing of cDNA probes directly onto a glass slide or other suitable substrate. A DNA clone is obtained for each gene of interest, purified, and amplified from a common vector by PCR using universal primers. The probes are robotically deposited in spots on the order of 50-200 µm in size. At this spacing, a density of, for example, approximately 10³ probes/cm² can be achieved.

Levels of the protein or peptide may be determined, for example, by using a "molecule binding to the protein or peptide" and preferably a "molecule specifically binding to the protein or peptide". A molecule binding to the protein or peptide designates a molecule which under known conditions occurs predominantly bound to the protein or peptide. A "molecule binding to the protein or peptide" may be one of the herein below described binding molecules, preferably inhibitors of the protein or peptide, such as antibodies, aptamers, etc. Levels of the protein or peptide may also be obtained by using Western Blot analysis, mass spectrometry analysis, FACS-analysis, ELISA, and immunohistochemistry. These techniques are non-limiting examples of methods which may be used to qualitatively, semi-quantitatively and/or quantitatively detect a protein or peptide.

Western blot analysis is a widely used and well-know analytical technique used to detect specific proteins or peptides in a given sample, for example, a tissue homogenate or body extract. It uses gel electrophoresis to separate native or denatured proteins or peptides by the length of the (poly)peptide (denaturing conditions) or by the 3-D structure of the protein (native/ non-denaturing conditions). The proteins or peptides are then transferred to a membrane (typically nitrocellulose or PVDF), where they are probed (detected) using antibodies specific to the target protein.

Also mass spectrometry (MS) analysis is a widely used and well-know analytical technique, wherein the mass-to-charge ratio of charged particles is measured. Mass spectrometry is used for determining masses of particles, for determining the elemental composition of a sample or molecule, and for elucidating the chemical structures of molecules, such as proteins, peptides and other chemical compounds. The MS principle consists of ionizing chemical compounds to generate charged molecules or molecule fragments and measuring their mass-to-charge ratios.

Fluorescence activated cell sorting (FACS) analysis is a widely used and well-known analytical technique, wherein biological cells are sorted based upon the specific light scattering of the fluorescent characteristics of each cell. Cells may be fixed in 4% formaldehyde, permeabilized with 0.2 % Triton-X-100, and incubated with a fluorophore-labeled antibody (e.g. mono- or polyclonal anti-HLA antibody).

Enzyme-linked immunosorbent assay (ELISA) is a widely used and well-known sensitive analytical technique, wherein an enzyme is linked to an antibody or antigen as a marker for the detection of a specific protein or peptide.

Immunohistochemistry (IHC) is the most common application of immunostaining. It involves the process of selectively identifying antigens (proteins) in cells of a tissue section by exploiting the principle of antibodies binding specifically to antigens in biological tissues. In combination with particular devices IHC can be used for quantitative *in situ* assessment of protein expression (for review Cregger et al. (2006) Arch Pathol Lab Med, 130:1026-1030). Quantitative IHC takes advantage of the fact that staining intensity correlates with absolute protein levels.

Methods for determining whether a subject responded to one or more of the tumor therapies and also for determining whether a subject that did respond to one or more of the tumor therapies are well-known in the art. Generally a tumor patient responds to a therapy if the tumor shrinks (in case of a solid tumor), if the number of tumor cells in a non-solid tumor (such as a blood cancer) or if the symptoms conferred by the tumorous disease are reduced or stay the same ("stabilizes"). Generally a tumor patient does not respond if the tumor worsens (e.g. increases it's size, increases its number of cells or in case the symptoms conferred by the tumorous disease aggravate) during treatment. In the connection with a response it is preferred that the tumor shrinks.

The definitive proof of the effectiveness of a therapy is improvement in clinical symptoms and survival whereas the definitive proof of the non-effectiveness of a therapy is worsening of clinical symptoms and ultimately the death of the subject. As part of this invention the disease specific survival is frequently being used, which is defined by the start of the treatment option under investigation until cancer specific death. Imaging, in particular of tumor lesions, is generally used to assess therapeutic effects earlier. Current response assessment is based primarily on changes in tumor size as measured by CT (computer tomography) or other anatomic imaging modalities, wherein shrinkage of the tumor size indicates a response. Also, imaging of tumor metabolism with PET (positron-emission-tomography) and the glucose analog ¹⁸F-FDG represents an attractive approach for assessing the effects of therapy objectively and quantitatively.

With respect to the evaluation of solid tumors it is preferred to use the response evaluation criteria in solid tumors (RECIST). RECIST is a set of rules that define when tumors in tumor patients ameliorate, stay the same, or worsen during treatment. The criteria were published in February 2000 by an international collaboration including the European Organisation for Research and Treatment of Cancer (EORTC), National Cancer Institute of the United States, and the National Cancer Institute of Canada Clinical Trials Group. Today, the majority of clinical trials evaluating cancer treatments for objective response in solid tumors use RECIST. These criteria were updated in 2009. With respect to the valuation of solid tumors it is also preferred to use the PET response criteria in solid tumors (PERCIST). PERCIST is an alternative set of rules that define when tumors in tumor patients ameliorate, stay the same, or worsen during treatment, using positron-emission-tomography (PET). These criteria were established in 2009.

The one or more subjects that responded or not responded, respectively, are with increasing preference at least 2, at least 5, at least 10 subjects, at least 25 subjects, and at least 50 subjects. Taking more than one subject has the advantage to bias for level differences among the patients with a response or no response, respectively.

Predetermined standards designate previously obtained values from one or more subjects that responded to one or more of the tumor therapies or one or more subjects that did not respond to one or more of the tumor therapies.

The increased level(s) of (B) and (B') are with increasing preference at least 1.1-fold, 1.2-fold, 1.3-fold, 1.4-fold, 1.5-fold, 2-fold, 3-fold, 4-fold increased as compared to the level of (A). The decreased level(s) of (B) and (B') are with increasing preference at least at least 1.1-fold, 1.2-fold, 1.3-fold, 1.4-fold, 1.5-fold, 2-fold, 3-fold, 4-fold decreased as compared to the level of (A). Substantially the same level(s) of (B) and (B') preferably differ (i.e. higher or lower) by less than 10%, more preferably less that 5% from the control or predetermined standard. For example, if the level in (A) is set to 100%, a substantially same level may be between less than 110% and more than 90% of the 100% control level.

As can be taken, from the examples herein below in was surprisingly found that the high expression level of membrane-bound HLA-G (exon 8 probe), soluble or membrane bound HLA-G (exon 3 probe), membrane-bound HLA-L (exon 7 probe), soluble HLA-H (exon 2/3 probe) and soluble HLA-J (exon 4/5 probe) in patients having bladder cancer and undergoing immune checkpoint therapy (anti-PD-1 or anti-PDL-1) is adversely associated with the survival of these patients. The higher the expression level of these HLA genes the more likely the patients died from the cancer within 2 years. It has to be taken into account, that posttranscriptional events may affect the membrane bound HLA isoforms. Therefore the determination of membrane bound HLA-G mRNA isoforms determined by exon 8 quantification may after proteolytic cleavage events following translation into the protein structures ultimatively result in soluble fragments of biological activity. However, the HLA mRNA expression levels were measured in tumor tissue samples that were obtained from the bladder cancer patients before the start of the immune checkpoint therapy. Hence, the data in the examples show that the subject's expression levels of the HLA-G, L, H and J genes or proteins can be used in order predict before an immune checkpoint therapy is started whether the subject will likely benefit from the treatment or not. While low expression levels are associated with superior disease specific survival high expression levels are associated with inferior disease specific survival.

It is believed that the predictive value of HLA-G, L, H and J expression levels shown in the examples for the survival of bladder cancer patients under immune checkpoint therapy is also applicable to other tumors and anti-tumor treatments, e.g. immunotherapies in general, chemotherapy, anti-hormonal therapy and anti-tyrosin therapy. This is because it can be assumed that high HLA-G, L, H and J expression levels help the tumor cells or a subpopulation of the tumor cells to escape the anti-tumor therapy, as any effective anti-cancer therapy results in tumor cell destruction and exposition of antigens to the immune system thereby demasking the tumor. Cellular strategies to reduce immune recognition as being conferred by HLA-G, L, H and J expression, therefore are of general importance not only for immune therapies but also for chemotherapy and/or anti-hormonal and/or tyrosin-kinase inhibitory therapy or any therapeutic combination thereof.

With regard to the sequences of membrane-bound human HLA-L, and soluble HLA-H, HLA-J and HLA-L it is of further note that it was surprisingly found herein that HLA-L, HLA-H and HLA-J were erroneously annotated as pseudogenes in the art. In fact, these genes are protein-coding and the expression of HLA-L, HLA-H and HLA-J can be detected in various cancers as is illustrated in the appended examples. Since HLA-L, HLA-H and HLA-J all were erroneously annotated in the art, HLA-L, HLA-H and HLA-J may be collectively described as a new HLA-group. In addition, the examples herein below show that high expression level of HLA-L, HLA-H and HLA-J in patients having bladder cancer is adversely associated with the survival of these patients. The higher the expression level of these HLA genes, the more likely the patients died from the cancer within 2 years. This body of evidence shows that the expression of HLA forms L, H and J is likely used by tumors as a mechanism of evading the immune system of the tumor patient. These genes and the encoded protein have a function and are not pseudogenes not encoding any functional protein.

In a preferred embodiment of the first aspect of the invention any one of SEQ ID NOs 2 and 4 to 6 is SEQ ID NO: 4 or 5, and any one of SEQ ID NOs 8 and 10 to 12 is SEQ ID NO: 11 or 12.

SEQ ID NOs 9 and 10 are the nucleic acid sequences encoding the soluble HLA forms of membrane-bound HLA-G and HLA-L, and SEQ ID NOs 11 and 12 are soluble HLA-H and HLA-J. SEQ ID NOs 3 to 6 are the corresponding amino acid sequences.

The data in the examples demonstrate on the basis of the HLA classes G, H, L and J that HLA genes and proteins can predict the response of a tumor patient to a tumor therapy as defined herein.

In a preferred embodiment of the first aspect of the invention, the method further comprises determining the mRNA expression level or the protein level of one or more selected from ErbB2, EGFR, CD20, CTLA4, IDO1, LAG3, TIM3, TIM-4, CXCL9, CXCL13, TIGIT, BTLA, CD137, OX40, VISTA, B7-H7, CD27, GITR, TGF-ß Signaling pathway, IL-15, PD-1 and PD-L1, preferably of PD-1 or PD-L1.

In connection with this preferred embodiment, it is to be understood that the mRNA expression level(s) or the protein level(s) are to be determined in the subject and are then compared to the respective control or predetermined standard from the known responders or non-responders and/or known survivors or non-survivors, just as explained herein above in connection with the HLA genes.

The mRNA expression level or the protein level of one or more selected from ERBB2, EGFR, CD20, CTLA4, IDO1, LAG3, TIM3, TIM-4, CXCL9, CXCL13, TIGIT, BTLA, CD137, OX40, VISTA, B7-H7, CD27, GITR, TGF-ß Signaling pathway, IL-15, PD-1 and PD-L1, preferably of PD-1 or PD-L1 are alone not of sufficient predictive value for determining whether a subject is likely to respond or not respond to a tumor therapy as defined herein, in particular an immune therapy and more particularly an checkpoint therapy. They may be useful in combination with the method of the present invention. Thus, the additional analysis of one or more of these level(s) is expected to further improve the predictive value of the method of the invention.

PD-1 (Programmed cell death protein 1, also known as CD279) is a protein on the surface of cells that has a role in regulating the immune system's response to the cells of the human body by down-regulating the immune system and promoting self-tolerance via suppressing T cell inflammatory activity.

PD-L1 (Programmed death-ligand 1, also known as CD274 or B7-H1) is a 40kDa type 1 transmembrane protein that has been speculated to play a major role in suppressing the immune system during particular events such as pregnancy, tissue allografts, autoimmune disease and other disease states such as hepatitis. Upregulation of PD-L1 may allow cancers to evade the host immune system. Importantly, PD-L1 might be expressed by tumour or non tumour cells such as macrophages etc.

ErbB2 (Receptor tyrosine-protein kinase erbB-2, also known as CD340 or proto-oncogene Neu) is a member of the human epidermal growth factor receptor (HER/EGFR/ERBB) family. Amplification or over-expression of this oncogene has been shown to play an important role in the development and progression of certain aggressive types of breast cancer.

EGFR (epidermal growth factor receptor, also known as HER1) is a transmembrane protein that is a receptor for members of the epidermal growth factor family (EGF family) of extracellular protein ligands.

CD20 is an activated-glycosylated phosphoprotein expressed on the surface of all B-cells beginning at the pro-B phase (CD45R+, CD117+) and progressively increasing in concentration until maturity. CD20 is the target of the monoclonal antibodies rituximab, ocrelizumab, obinutuzumab, ofatumumab, ibritumomab tiuxetan, tositumomab, and ublituximab, which are all active agents in the treatment of all B cell lymphomas, leukemias, and B cell-mediated autoimmune diseases.

CTLA4 (cytotoxic T-lymphocyte-associated protein 4, also known as CD152), is a protein receptor that, functioning as an immune checkpoint (or checkpoint inhibitor), downregulates immune responses. CTLA4 is constitutively expressed in regulatory T cells but only upregulated in conventional T cells after activation - a phenomenon which is particularly notable in cancers.

IDO1 (Indoleamine-pyrrole 2,3-dioxygenase) is a heme-containing enzyme. IDO1 has been implicated in immune modulation through its ability to limit T-cell function and engage mechanisms of immune tolerance. IDO becomes activated during tumor development, helping malignant cells to escape eradication by the immune system.

LAG3 (Lymphocyte-activation gene 3, also known as CD223) is a cell surface molecule with diverse biologic effects on T cell function. It is an immune checkpoint receptor and as such is the target of various drug development programs by pharmaceutical companies seeking to develop new treatments for cancer and autoimmune disorders.

TIM-3 (T-cell immunoglobulin and mucin-domain containing-3, also known as Hepatitis A virus cellular receptor 2 (HAVCR2)) mediates the CD8+ T-cell exhaustion. TIM-3 has also been shown as a CD4+ Th1-specific cell surface protein that regulates macrophage activation and enhances the severity of experimental autoimmune encephalomyelitis in mice.

TIM-4 (T-cell immunoglobulin and mucin-domain containing-4) is a phosphatidylserine receptor that enhances the engulfment of apoptotic cells. TIM-4 is involved in regulating T-cell proliferation and lymphotoxin signaling.

CXCL9 (chemokine (C-X-C motif) ligand 9) is a small cytokine belonging to the CXC chemokine family that is also known as Monokine induced by gamma interferon (MIG). CXCL9 is a T-cell chemoattractant, which is induced by IFN-γ.

CXCL13 (chemokine (C-X-C motif) ligand 1, also known as B lymphocyte chemoattractant (BLC) or B cell-attracting chemokine 1 (BCA-1)) is a small chemokine belonging to the CXC chemokine family. As its name suggests, this chemokine is selectively chemotactic for B cells belonging to both the B-1 and B-2 subsets, and elicits its effects by interacting with chemokine receptor CXCR5.

TIGIT also called T cell immunoreceptor with Ig and ITIM domains) is an immune receptor present on some T cells and Natural Killer Cells (NK). It is also identified as WUCAM and Vstm3. TIGIT and PD-1 have been shown to be overexpressed on tumor antigen-specific (TA-specific) CD8+ T cells and CD8+ tumor infiltrating lymphocytes (TILs) from individuals with melanoma.

BTLA (B- and T-lymphocyte attenuator, also known as CD272) expression is induced during activation of T cells, and BTLA remains expressed on Th1 cells but not Th2 cells. BTLA activation inhibits the function of human CD8+ cancer-specific T cells.

CD137 is also known as tumor necrosis factor receptor superfamily member 9 (TNFRSF9), 4-1BB and induced by lymphocyte activation (ILA). The best characterized activity of CD137 is its costimulatory activity for activated T cells. Crosslinking of CD137 enhances T cell proliferation, IL-2 secretion, survival and cytolytic activity. Further, it can enhance immune activity to eliminate tumors.

Ox40 (also known as tumor necrosis factor receptor superfamily, member 4 (TNFRSF4) and CD134) is a secondary co-stimulatory immune checkpoint molecule, expressed after 24 to 72 hours following activation; its ligand, OX40L, is also not expressed on resting antigen presenting cells, but is following their activation. Expression of OX40 is dependent on full activation of the T cell; without CD28, expression of OX40 is delayed and of fourfold lower levels.

VISTA (V-domain Ig suppressor of T cell activation) is a type I transmembrane protein that functions as an immune checkpoint. VISTA can act as both a ligand and a receptor on T cells to inhibit T cell effector function and maintain peripheral tolerance.

B7-H7 (also known as human endogenous retrovirus-H long terminal repeat associating 2 (HHLA2)) is a B7 family member that regulates human T-cell functions. B7-H7 was previously known as with unidentified function. B7-H7 has been identified as a specific ligand for human CD28H. The B7-H7-CD28H pathway strongly promoted CD4+ T-cell proliferation and cytokine production via an AKT-dependent signaling cascade in the presence of TCR signaling, suggesting B7-H7 comprises a new co-stimulatory pathway. The first IgV domain of B7-H7, which presumably binds to a putative receptor, shows the highest homology to other B7 family members.

CD27 is required for generation and long-term maintenance of T cell immunity. It binds to ligand CD70, and plays a key role in regulating B-cell activation and immunoglobulin synthesis.

GITR (glucocorticoid-induced TNFR-related protein, also known as tumor necrosis factor receptor superfamily member 18 (TNFRSF18) and activation-inducible TNFR family receptor (AITR)) has been shown to have increased expression upon T-cell activation, and it is thought to play a key role in dominant immunological self-tolerance maintained by CD25+/CD4+ regulatory T cells. Knockout studies in mice also suggest the role of this receptor is in the regulation of CD3-driven T-cell activation and programmed cell death.

The transforming growth factor beta (TGFβ) signaling pathway is involved in many cellular processes in both the adult organism and the developing embryo including cell growth, cell differentiation, apoptosis, cellular homeostasis and other cellular functions. In spite of the wide range of cellular processes that the TGFβ signaling pathway regulates, the process is relatively simple. TGFβ superfamily ligands bind to a type II receptor, which recruits and phosphorylates a type I receptor. The type I receptor then phosphorylates receptor-regulated SMADs (R-SMADs) which can now bind the coSMAD SMAD4. R-SMAD/coSMAD complexes accumulate in the nucleus where they act as transcription factors and participate in the regulation of target gene expression.

IL-15 (Interleukin-15) is a cytokine with structural similarity to Interleukin-2 (IL-2). Like IL-2, IL-15 binds to and signals through a complex composed of IL-2/IL-15 receptor beta chain (CD122) and the common gamma chain (gamma-C, CD132). IL-15 is secreted by mononuclear phagocytes (and some other cells) following infection by virus(es). This cytokine induces cell proliferation of natural killer cells; cells of the innate immune system whose principal role is to kill virally infected cells.

In accordance with a preferred more embodiment of the first aspect of the invention the immune checkpoint inhibitor is selected from the group consisting of Nivolumab, Pembrolizumab, Cemiplimab, and.

Nivolumab (marketed as Opdivo) is an anti-PD-1 monoclonal antibody and is used to treat cancer. Pembrolizumab (formerly MK-3475 and lambrolizumab, trade name Keytruda) and Cemiplimab are further anti-PD-1 antibodies and used to treat cancer.

Atezolizumab is an antibody against the protein programmed cell death-ligand 1 (PD-L1) and used for cancer immunotherapy. Durvalumab and Avelumab are further antibodies against PD-L1 being useful for the treatment of cancer.

The present invention relates in a second aspect to a method for preparing a kit for predicting whether a subject having urothelial cancer responds to an anti-PD-1 or anti-PD-L1 immune checkpoint therapy, wherein the method comprises combining means for the detection of the level(s) of at least one nucleic acid molecule as defined in the first aspect of the invention and/or at least one protein or peptide as defined in the first aspect of the invention, and instructions how to use the kit.

The kit to be prepared implements a/the means required for conducting the invention of the invention in the format of a kit. For this reason the definitions and preferred embodiments provided herein above in connection with the first aspect of the invention are equally applicable to the kit of the invention.

A/the means for the detection and/or quantification of the nucleic acid molecule as exemplified as part of this invention may be one or more of the primer and probes as shown herein below in Table 1. However, any detection module being capable of quantifying nucleic acids such as arrays, NGS or other molecular systems would be appropriate as part of this invention. A/the means for the detection of the protein or peptide are preferably an antibody and/or protein binder and/or peptide binder as described herein above. For detection and/or quantification the antibody and/or protein binder and/or peptide binder may be labelled, e.g. by a fluorescent dye or a radiolabel. Examples of fluorescent dyes and radiolabels are also described herein above.

The various components of the kit may be packaged into one or more containers such as one or more vials. The vials may, in addition to the components, comprise preservatives or buffers for storage. The kit may comprise instructions how to use the kit, which preferably inform how to use the components of the kit for predicting whether a subject having a tumor responds to a tumor therapy as defined herein.

In a preferred embodiment of the second aspect of the invention the means comprise primer pairs and optionally a hydrolysis probe or other labelled primer or probe detection approaches for target sequence quantitation known to persons skilled in the art such as scorpion primers, FRET-probes or molecular beacons used for the sequence-specific detection of at least one nucleic acid molecule as defined herein above.

The primer pairs and optionally a hydrolysis probe are generally used for the specific detection of at least one nucleic acid molecule as defined herein above in a real time quantitative PCR a described herein above. Preferred primer pairs and hydrolysis probes are shown herein below in Table 1.

The hydrolysis probe designates the above-discussed sequence-specific DNA probe consisting of an oligonucleotide that is labelled with a fluorescent reporter which permits detection only after hybridization of the probe with its complementary sequence (e.g. a TaqMan probe). In more detail, hydrolysis probes are dual-labelled oligonucleotides. The 5' end of the oligonucleotide is labelled with a fluorescent reporter molecule while the 3' end is labelled with a quencher molecule. The sequence of the probe is specific for a region of interest in the amplified target molecule. The hydrolysis probe is designed so that the length of the sequence places the 5' fluorophore and the 3' quencher in close enough proximity so as to suppress fluorescence. During the extension phase of the PCR cycle the DNA polymerase synthesizes the complementary strand downstream of the PCR primers. When extension reaches the bound hydrolysis probe the 5'-3' exonuclease activity of the DNA polymerase degrades the hydrolysis probe. Cleavage of the hydrolysis probe separates the fluorescent reporter molecule from the rest of the probe allowing the reporter molecule to fluoresce.

The Figures show.

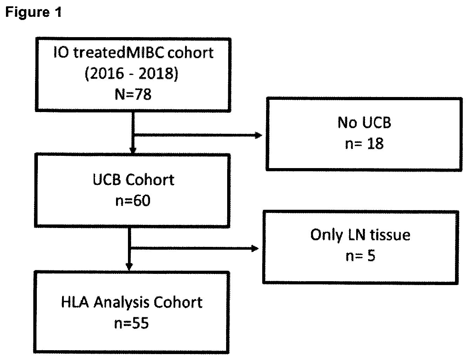

**Figure 1****.** Consort Diagram of advanced or metastatic urothelial cancer cohort. After exclusion of formalin-fixed paraffin-embedded (FFPE) blocks with insufficient and/or lymphnode tissues, tissues of 55 patients were available for analysis.

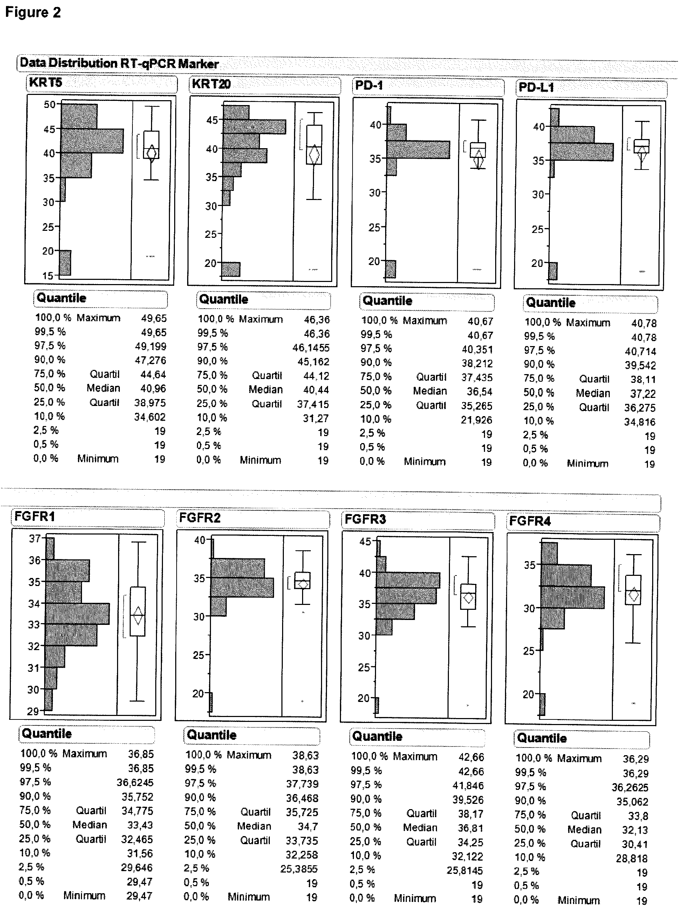

**Figure 2****.** Data distribution of luminal and basal subtype markers, check point target genes and FGFR1 to 4 gene expression as determined by RT-qPCR from tissues from muscle invasive bladder cancer patients.

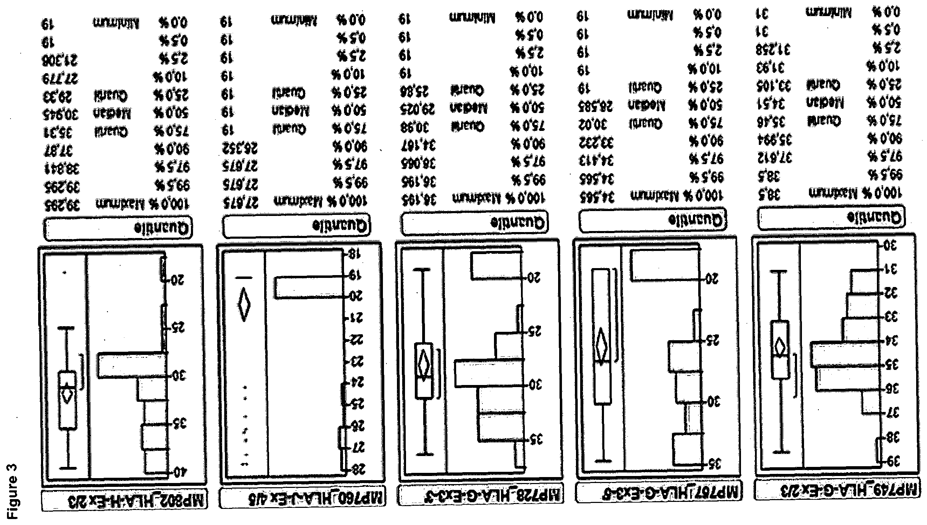

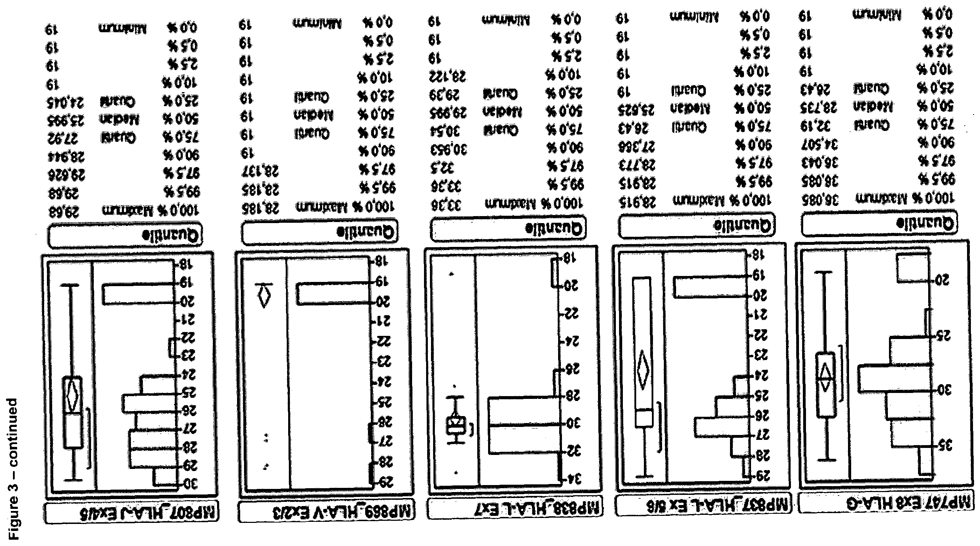

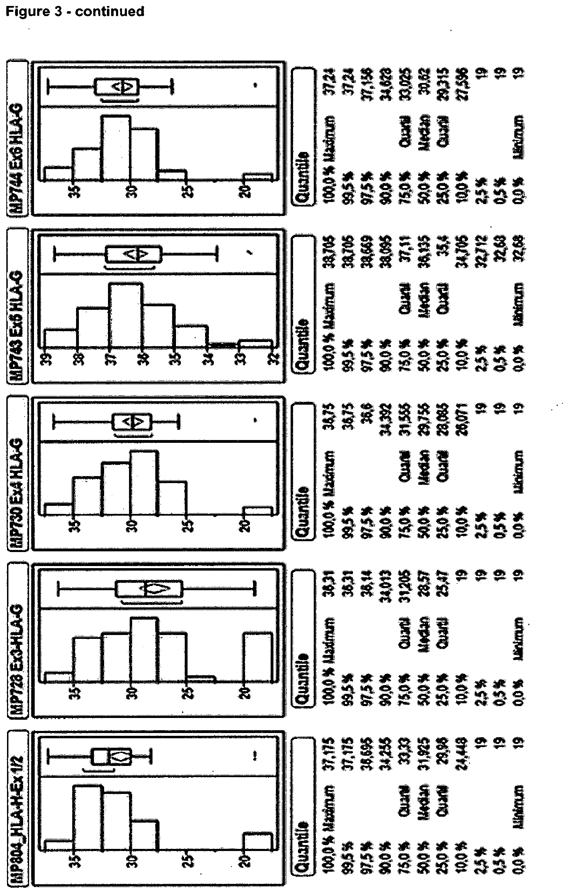

**Figure 3****.** Quantification of HLA -G, -H, -J, -L, - V mRNA expression by RT-qPCR assay of distinct exon regions. Relative mRNA expression is determined by the 40-DCT method using CALM2 as reference gene. The higher the 40-DCT value, the higher the gene expression.

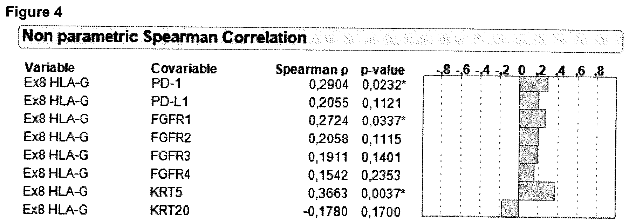

**Figure 4****.** Intergene spearman correlation of luminal and basal subtype markers, check point target genes, FGFR1 to 4 genes and exon 8 mRNA expression analysis of HLA-G, as determined by RT-qPCR from tissues from muscle invasive bladder cancer patients.

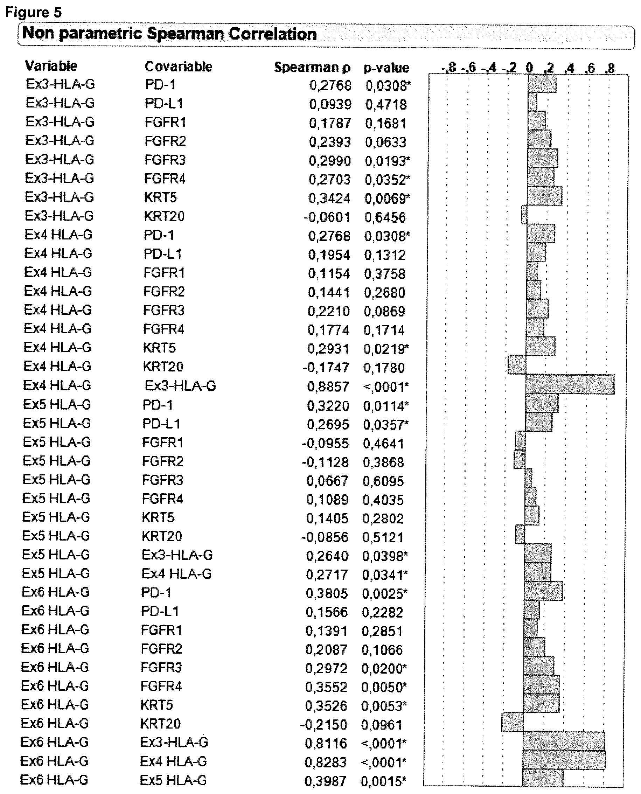

**Figure 5****.** Intergene spearman correlation of luminal and basal subtype markers, check point target genes, FGFR1 to 4 genes and HLA-G exon 3 to 6 mRNA expression analysis as determined by RT-qPCR in tissues from muscle invasive bladder cancer patients (n=61).

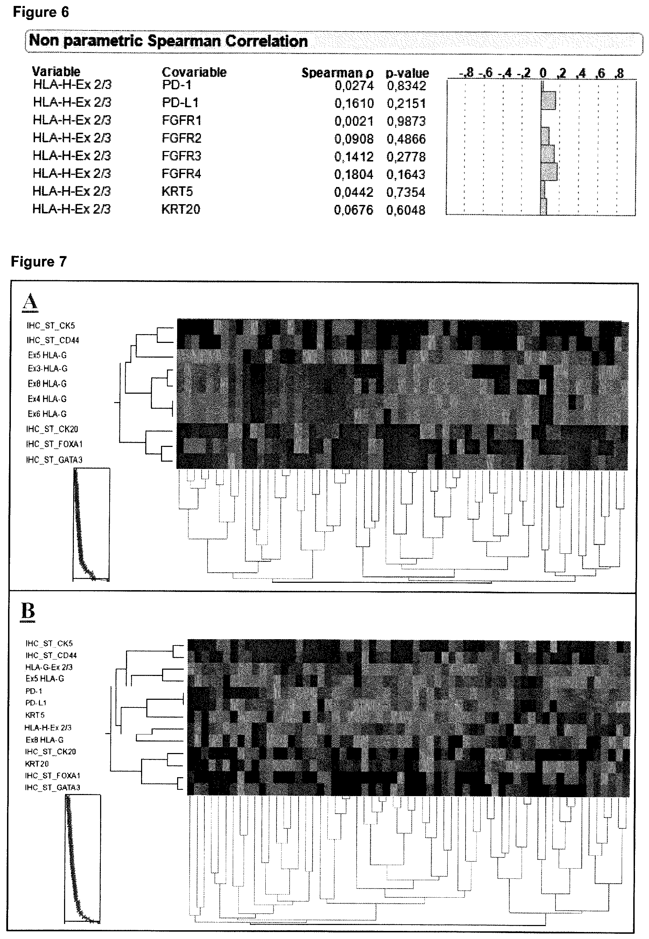

**Figure 6****.** Correlation of HLA-H mRNA expression in urothelial cancer patients with FGFR receptors, PD-1, PD-L1 and markers for basal and luminal cell type.
**Figure 7****.** Cluster analysis of HLA genes with immune histological and molecular assessed urothelial markers. Red highlights high gene expression, whereas blue depict low gene expression. Genes are depicted on the left side of the cluster analysis. Each column represents a cystectomy UBC sample form a patient

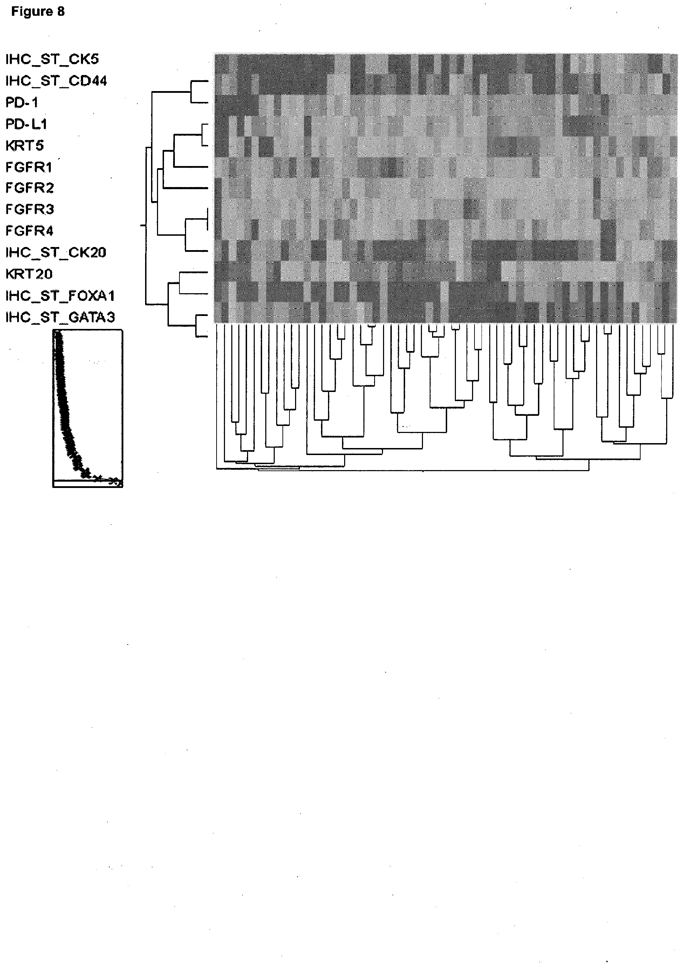

**Figure 8****.** Cluster analysis of FGF receptor genes with PD-1, PD-L1 and basal and luminal markers. Red highlights high gene expression, whereas blue depict low gene expression. Genes are depicted on the left side of the cluster analysis. Each column represents a cystectomy UBC sample form a patient

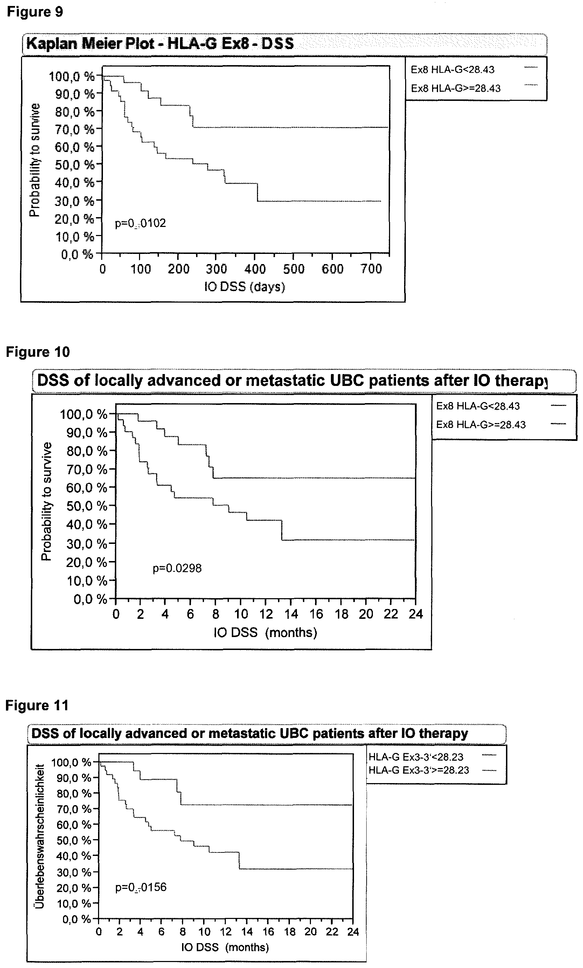

**Figure 9****.** Kaplan Meier Plot displaying disease specific survival (DSS) probability from muscle invasive bladder cancer patients based on stratification by HLA-G exon 8 expression as quantified by RT-qPCR assay. Relative mRNA expression is determined by the 40-DCT method using CALM2 as reference gene.
**Figure 10****.** Kaplan Meier Plot displaying disease specific survival (DSS) probability from muscle invasive bladder cancer patients having locally advanced or metastatic UBC (n=57) based on stratification by HLA-G exon 8 expression as quantified by RT-qPCR assay. Relative mRNA expression is determined by the 40-DCT method using CALM2 as reference gene.
**Figure 11****.** Kaplan Meier Plot displaying disease specific survival (DSS) probability from muscle invasive bladder cancer patients having locally advanced or metastatic UBC (n=57) based on stratification by HLA-G exon 3 expression as quantified by RT-qPCR assay. Relative mRNA expression is determined by the 40-DCT method using CALM2 as reference gene.

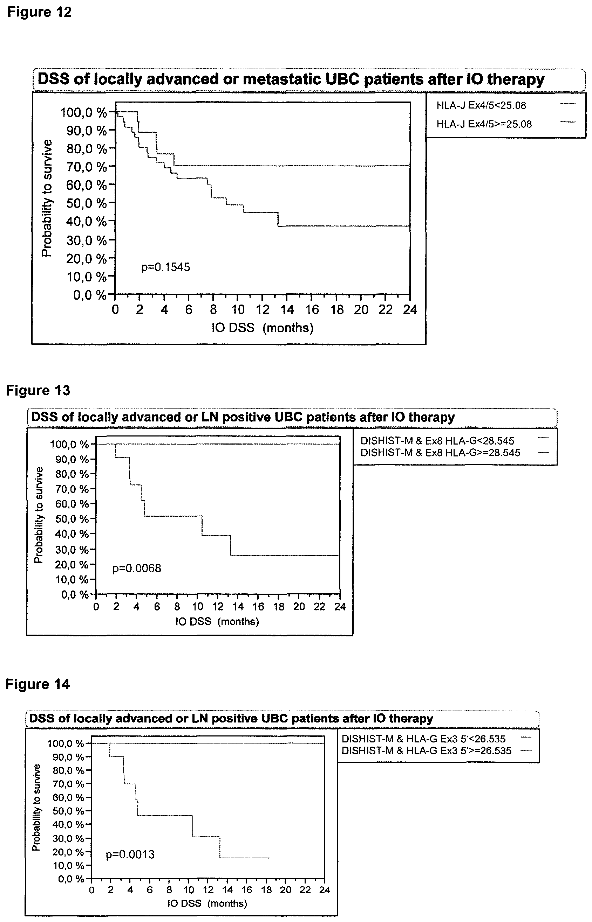

**Figure 12****.** Kaplan Meier Plot displaying disease specific survival (DSS) probability from muscle invasive bladder cancer patients having locally advanced or metastatic UBC (n=57) based on stratification by HLA-J Exon 4/5 expression as quantified by RT-qPCR assay. Relative mRNA expression is determined by the 40-DCT method using CALM2 as reference gene.
**Figure 13****.** Kaplan Meier Plot displaying disease specific survival (DSS) probability from muscle invasive bladder cancer patients having locally advanced or lymph node positive UBC (n=20) based on stratification by HLA-G exon 8 expression as quantified by RT-qPCR assay. Relative mRNA expression is determined by the 40-DCT method using CALM2 as reference gene.
**Figure 14****.** Kaplan Meier Plot displaying disease specific survival (DSS) probability from muscle invasive bladder cancer patients having locally advanced or lymph node positive UBC (n=20) based on stratification by HLA-G exon 3 expression as quantified by RT-qPCR assay. Relative mRNA expression is determined by the 40-DCT method using CALM2 as reference gene.

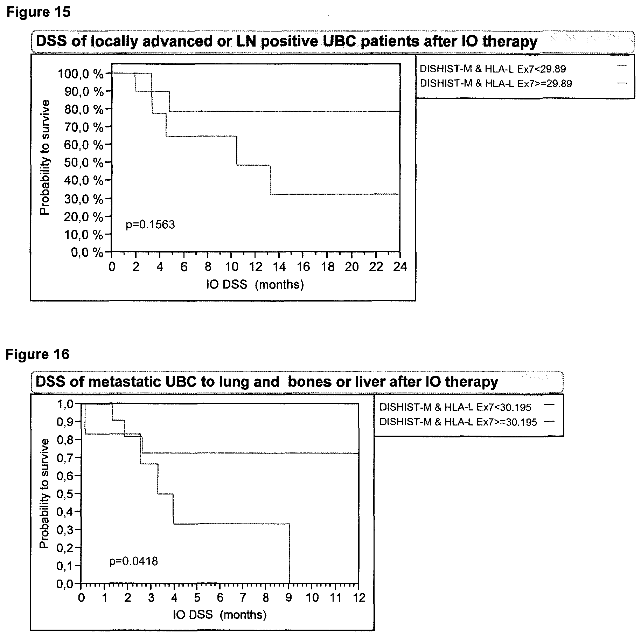

**Figure 15****.** Kaplan Meier Plot displaying disease specific survival (DSS) probability from muscle invasive bladder cancer patients having locally advanced or lymph node positive UBC (n=19) based on stratification by HLA-L exon 7 expression as quantified by RT-qPCR assay. Relative mRNA expression is determined by the 40-DCT method using CALM2 as reference gene.
**Figure 16****.** Kaplan Meier Plot displaying disease specific survival (DSS) probability from muscle invasive bladder cancer patients having metastasized to lung and bones or liver (n=17) based on stratification by HLA-L exon 7 expression as quantified by RT-qPCR assay. Relative mRNA expression is determined by the 40-DCT method using CALM2 as reference gene.

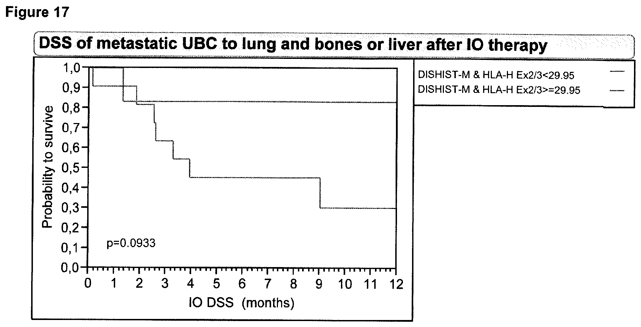

**Figure 17****.** Kaplan Meier Plot displaying disease specific survival (DSS) probability from muscle invasive bladder cancer patients having metastasized to lung and bones or liver (n=17) based on stratification by HLA-H exon 2/3 expression as quantified by RT-qPCR assay. Relative mRNA expression is determined by the 40-DCT method using CALM2 as reference gene.

The examples illustrate the invention.

### Example 1:

### EXAMPLES

### Example 1: HLA profiling in advanced, chemotherapy refractory urothelial cancer

Transurethral resection (TUR) biopsies and cystectomy samples from primary tumors being refractory to chemotherapy and thereafter undergoing first or second line immuneoncology ("IO") treatment by PD-1 and PD-L1 checkpoint inhibitor drugs (i.e. Atezolizumab, Nivolumab and Pembrolizumab) were analyzed for HLA expression and associated with histopathological and molecular parameters as well as response to IO treatment and disease specific survival after IO.

Seventy-two newly diagnosed patients with histologically confirmed urothelial cancer, including bladder cancer and upper urothelial tract carcinoma were enrolled in the study between 2016 and 2018. Nivolumab, Pemprolizumab and Atezumab were given as 1st, 2nd and 3rd line mono-treatment according to approved instructions. All hematoxylin-eosin (HE) stained tumor tissue sections from samples of the cohort were evaluated and classified according to TNM-classification (2017) of the UICC by two uro-pathologists. Rare histological variants were classified according to the World Health Organization (WHO 2016) classification of genitourinary tumors. After central histopathological review 18 tissues were excluded for not having sufficient tumor material or not being urothelial cancer. From 5 patients only lymphnode tissue was available and therefore excluded from primary analysis of prognostic and/or predictive effects of HLA gene expression (see Figure 1; Consort Diagram). Data base closure for clinical data was done on October 16^{th} 2018 in conjunction with a parallel FDA submission.

For mRNA detection, RNA was extracted from FFPE tissue from TUR biopsies, cystectomy and corresponding mapping bladder tissue using commercial kits (Xtract, Stratifyer). For each reaction, 2,5 µl total RNA extracted from FFPE sections were mixed with 2.5 µl assay-mix, 2.5 µl enzyme-mix and 2,5 µl water in one well of a 96-well-optical reaction plate. Measurements of the PCR reaction were done according to the instructions of the manufacturer with a Versant kPCR Cycler (Siemens) or a Light Cycler 480 (Roche) under appropriate conditions (5 min 50°C, 1 cycle; 20 s 95°C, 1 cycle; 15 s 95°C; 1 min 60°C, 40 cycles). The relative mRNA expression was associated with response to IO treatment determined based on RECIST (Response Evaluation Criteria in Solid Tumors) criteria as assessed at the individual sites and with disease specific survival as determined from start of IO treatment to cancer specific death. Partition testing using biostatistical JMP SAS 9.0.0 (SAS, Cary, North Carolina, USA) were performed to evaluate the possible differences in response to IO treatment.

For a detailed analysis of gene expression by RT-qPCR methods, primers flanking the region of interest and a fluorescently labeled probe hybridizing in-between were utilized. Target-specific primers and probes were selected using the NCBI primer designing tool (www.ncbi.nlm.nih.go). RNA-specific primer/probe sequences were used to enable RNA-specific measurements by locating primer/probe sequences across exon/exon boundaries. Furthermore, primers/probes were selected not to bind to sequence regions with known polymorphisms (SNPs). In case multiple isoforms of the same gene existed, primers were selected to amplify all relevant or selected splice variants as appropriate. All primer pairs were checked for specificity by conventional PCR reactions. After further optimization of the primers/probes, the primers and probes listed in Table 1 gave the best results. These primers/probes are superior to primers/probes known from the prior art, e.g., in terms of specificity and amplification efficiency. To standardize the amount of sample RNA, the CALM2 was selected as reference gene, since they were not differentially regulated in the samples analyzed. TaqMan^{®} validation experiments were performed showing that the efficiencies of the target and the control amplifications were approximately equal, which is a prerequisite for the relative quantification of gene expression by the comparative ΔCT method.

**Table 1. Used primers and probes for HLA mRNA quantitation**

| Gen | For_Primer | Probe | Rev-Primer |
|---|---|---|---|
| HLA-G-Ex3 | GGCCGGAGTATTGGGAAGA | CAAGGCCCACGCACAGACTGACA | GCAGGGTCTGCAGGTTCATT |
| HLA-G Ex4 | CTGCGGCTCAGATCTCCAA | CGCAAGTGTGAGGCGGCCAAT | CAGGTAGGCTCTCCTTTGTTCAG |
| HLA-G Ex5 | CACCACCCTGTCTTTGACTATGAG | ACCCTGAGGTGCTGGGCCCTG | AGTATGATCTCCGCAGGGTAGAAG |
| HLA-G Ex6 | CATCCCCATCATGGGTATCG | TGCTGGCCTGGTTGTCCTTGCA | CCGCAGCTCCAGTGACTACA |
| HLA-G Ex8 | GACCCTCTTCCTCATGCTGAAC | CATTCCTTCCCCAATCACCTTTCCTGTT | CATCCCAGCCCCTTTTCTG |
| HLA-G Ex3-5 | TTCATCGCCATGGGCTACG | CGACACGCAGTTCGTGCGGTTC | ATCCTCGGACACGCCGAGT |
| HLA-G Ex2/3 | CCGAACCCTCTTCCTGCTGC | CGAGACCTGGGCGGGCTCCC | GCGCTGAAATACCTCATGGA |
| HLA-H Ex 2/3 | GAGAGAACCTGCGGATCGC | AGCGAGGGCGGTTCTCACACCATG | CCACGTCGCAGCCATACAT |
| HLA-H | GAGAGAACCTGCGGATCGC | ACCAGAGCGAGGGCGGTTCTCACAC | CGGGCCGGGACATGGT |
| KRT5 | CGCCACTTACCGCAAGCT | TGGAGGGCGAGGAATGCAGACTCA | ACAGAGATGTTGACTGGTCCAACTC |
| KRT20 | GCGACTACAGTGCATATTACAGAC AA | TTGAAGAGCTGCGAAGTCAGATTAAGGATGCT | CACACCGAGCATTTTGCAGTT |
| PD-1 | GGCCAGCCCCTGAAGGA | ACCCCTCAGCCGTGCCTGTGTTC | GGAAATCCAGCTCCCCATAGTC |
| PDL1 | TGGCATCCAAGATACAAACTCAA | CAAAGTGATACACATTTGGAGGAGACGTAA | TTGAAGATCAGAAGTTCCAATGCT |
| CALM2 | GAGCGAGCTGAGTGGTTGTG | TCGCGTCTCGGAAACCGGTAGC | AGTCAGTTGGTCAGCCATGCT |
| HLA-L Ex2/3 | CCTGCTCCGCTATTACAACCA | CGAGGCCGGTATGAACAGTTCGCCTA | CGTTCAGGGCGATGTAATCC |
| HLA-L Ex5/6 | GCTGTGGTTGCTGCTGCG | AGAAAAGCTCAGGCAGCAATTGTGCTCAG | CATAGTCCTCTTTACAAGTATCATGAGA TG |
| HLA-L Ex 7 | TCCTCTTCTGCTCAGCTCTCCTA | CTCTCCCTTCCCTGAGTTGTAGTAATCCTAGCA CT | GCTTTATAGATCCATGAGTTTGCATTA |
| HLA-J Ex4/5 | CAAGGGGCTGCCCAAGC | CATCCTGAGATGGGTCACACATTTCTGGAA | CCTCCTAGTCTTGGAACCTTGAGAAGT |

The determination of luminal and basal subtypes in the UC cohort by RT-qPCR revealed a broad dynamic range of KRT5 and KRT20 mRNA ranging from 40-DCT values of 19 to 48 in similar ranges. The mRNA expression of PD-1 and PD-L1 ranged from 19 to 41. The dynamic range for the FGFR genes differed markedly within the FGFR family. The relative FGFR1 mRNA ranged from 29 to 37, FGFR2 mRNA from 19 to 39, FGFR3 mRNA from 19 to 43 and FGFR4 mRNA from 19 to 36 (figure 2).

In addition to the mRNA expression analysis of luminal and basal markers, PD-1, PD-L1 and the FGFR family, the expression profile of classical HLAs as well as exon expression of HLA genes and pseudogenes have been carried out (Figure 3).

Non-parametric spearman correlation of the FGFR genes 1-4, PD-1, PD-L1, basal and luminal markers as well as exon 8 HLA primer sets reveals a strong and significant correlation of PD-1 (spearman rho 0.2904, p=0,0232) in urothelial tumors expressing HLA-G exon 8. Besides PD-1, high FGFR1 (spearman rho 0.2724, p=0.0337) expression is also associated with HLA-G exon 8 expression. However, no significant correlation could be observed with any HLA for the luminal like urothelial carcinomas (figure 4).

Surprisingly, spearman correlation of luminal and basal subtype markers, check point target genes, FGFR1 to 4 genes with remaining HLA-G exons reveals a strong and significant association of HLA-G with the check point marker PD-1 as indicated by an similar high coexpression with all HLA-G exons (PD-1 in exon 3 3'end: spearman rho 0.2768, p=0.0308; PD-1 in exon 4: spearman rho 0.2768, p=0.0308; PD-1 in exon 5: spearman rho 0.3220, p=0.0114; PD-1 in exon 6: spearman rho 0.3805, p=0.0025)(figure 5). These interesting finding could only be confirmed in HLA-G exon 5 for PD-L1 (spearman rho 0.2695, p=0.0357). For exon 3 3' end, high significant correlation can also be observed for FGF receptor 3 (spearman rho 0.2990, p=0.0193) and 4 (spearman rho 0.2703, p=0.0352). This association could not be determined for exon 4, though exon 4 expression was associated with high mRNA expression of the basal cell marker KRT5 (spearman rho 0.2931, p=0.0219). The basal marker KRT5 (spearman rho 0.3526, p=0.0053) showed also significant correlation with HLA-G exon 6. In addition, FGF receptors 3 (spearman rho 0.2972, p=0.0200) and 4 (spearman rho 0.3552, p=0.0050) show also significant correlations with HLA-G exon 6 in mRNA expression.

In addition, non parametric Spearman correlation analysis of luminal and basal subtype markers, PD1, PD-L1 and FGFR1 to 4 genes has also been done for HLA-H (figure 6). However, no correlation between HLA-H expression and luminal or basal markers or check point inhibitors could be observed.

Further, cluster analysis of FGF receptor genes with PD-1, PD-L1 and basal and luminal markers was carried out. The analysis revealed that PD-1 and PD-L1 expression occurs in rather basal urothelial cancer subtypes. In addition, FGFR1 mRNA is also higher expressed in Cytokeratin 5 positive tumors, whereas FGF receptors 2 to 4 show higher expression in rather luminal urothelial cancer subtype.

Cluster analysis of HLA genes with immune histological assessed urothelial cancer markers, reveal that HLA-G expression mainly occurs in basal urothelial cancer subtypes (IHC_ST_CK5), The basal urothelial cancer subtype can further be divided by HLA mRNA expression. Some basal tumor subtypes show a high HLA-G expression (figure 7 A). Finally, cluster analysis of HLA Exon 8 expression with immune histological cell and subclassification markers (CK5, CD44, CK20, FOXA1, GATA3) PD-1, PD-L1 as well as HLA-H expression was performed. The analysis revealed that HLA-G Exon 5 and Exon 8 expression and HLA-H expression as well as PD-1, PD-L1 can rather be assigned to the basal subtype. However, HLA-G, HLA-H and PD-1 and PD-L1 expression could also be observed in luminal urothelial tumors (figure 7 B). In addition, in silico promoter analysis revealed several estrogene and response elements (ERE) as well as a progesterone response element (PRE) in the HLA-G gene. This indicates the important potential of HLA-G expression not only in basal but also in luminal cancer subtypes. Since mRNA exon and exon/exon junction expression varies within the luminal and basal cancer subtypes single exon expression and exon/exon junction analysis as a stratification tool should be applied in basal as well as luminal urothelial cancer subtypes. Surprisingly, in silico analysis of the HLA-H promoter region also revealed several estrogen response elements. Together with the cluster analysis, this underlines the important role of the pseudogene HLA-H as a further stratification tool in urothelial cancer. As depicted in figure 8, further cluster analysis of FGF receptor genes has been carried out with PD-1, PD-L1 and basal and luminal markers. The analysis proved, that PD-1 and PD-L1 expression occurs in rather basal urothelial cancer subtypes. In addition, FGFR1 mRNA is also higher expressed in Cytokeratin 5 positive tumors, whereas FGF receptors 2 to 4 show higher expression in rather luminal urothelial cancer subtype. This demonstrates the representativity of the cohort analyzed for HLA gene interactions.

### Example 2: Exon expression of different HLA genes in urothelial cancer as marker for disease specific survival (DSS)

To determine the predictive value of HLA gene expression in bladder cancer tissues of advanced or metastatic urothelial cancer patients undergoing immune-oncological checkpoint therapy (IO therapy) (i.e. Atezolizumab, Nivolumab or Pembrolizumab) were assessed based on detailed clinical follow up data, which comprised i.a. WHO grading, primary metastatic sites, start of IO treatment, time point of cancer specific death or last contact date. The immune-oncological disease specific survival was calculated from start of IO therapy to cancer specific death or last contact and censored respectively.

As depicted in figure 9 relevance of changes of HLA-G mRNA expression on disease specific survival (DSS) of urothelial cancer patients was analyzed. When taking all available tissues including metastatic lymphnodes into account (n=60) Kaplan Meier analysis revealed that an increased HLA-G Exon 8 mRNA expression above 28.43 40-DCT values indicated worse disease specific survival (p=0.0102).

However, to exclude non cancer associated effects of HLA expression by non-tumor-associated lymphocytes in the lymph nodes, the metastatic lymph node tissues were excluded from the subsequent analysis, leaving 57 samples for survival analysis as depicted in Figure 1. As depicted in Figure 10 high HLA-G Exon 8 mRNA expression (>= 28.43) was significantly associated with inferior disease specific survival with HLA-G Exon 8 positive patients having a survival probability of 35% after 2 years, while HLA-G Exon 8 negative patients had a survival probability of 65% after 2 years (p=0,0298)..

As the examined HLA-G specific Exon 8 region is not translated into protein further confirmatory analysis has been performed by determining the Exon 3 region of HLA-G, which is part of the translated region close to the signal peptide of HLA-G. As depicted in Figure 11 high HLA-G Exon 3 mRNA expression (>= 28.23) was significantly associated with inferior disease specific survival with HLA-G Exon 3 positive patients having a survival probability of 30% after 2 years, while HLA-G Exon 8 negative patients had a survival probability of 70% after 2 years (p=0.0156).

Next the prognostic value of other HLA genes in the total cohort was analyzed. Special focus has been on currently classified "pseudogenes" as exemplified for HLA-J, H, V or L. As depicted in Figure 12 high HLA-J Exon 4/5 mRNA expression (>= 25.08) was associated with inferior disease specific survival with 36 HLA-J Exon 4/5 positive patients having a survival probability of 35% after 2 years, while 19 HLA-J Exon 4/5 negative patients had a survival probability of 70% after 2 years.

To further elucidate the relevance of HLA-G expression on survival after IO therapy the analysis was further specified by analyzing only primary tumor tissue and in addition also taking the primary metastatic site into account. This is based on initial findings that IO therapy has differential effects depending on the site of metastasis with e.g. visceral metastasis into the liver being less effective, probably due to the fact that PD1 positive T-cells are being excluded from the liver in metastatic urothelial cancer patients independent of classical checkpoint mechanisms (Eckstein M, Sikic D, Strissel PL, Erlmeier F. Evolution of PD-1 and PD-L1 Gene and Protein Expression in Primary Tumors and Corresponding Liver Metastases of Metastatic Bladder Cancer. Eur Urology 2018.). Therefore the patients were grouped according to the first manifestation of metastasis with local advancement, locoregional lymph nodes or extraregional retroperitoneal lymph nodes being categorized as 0 or 0,5, respectively, while dissemination into the bones, liver, lung, lung and bone or lung and liver were categorized with increasing indices (1, 2, 3, 4, 5; respectively). For this analysis 54 datasets from primary tumor tissues with sufficient clinical date and primary tumor tissue material were available, with 19 patients having local advancement or lymph node metastasis, while 17 patients had initially metastasized to bone or liver and 18 patients having metastasized with lung involvement either as singular site or in combination with bone or liver involvement, while all of them had been treated with IO drugs and predominantly > 1^{st} line setting (74%).

In urothelial bladder cancer patients having advanced or lymph node positive disease high mRNA expression of HLA-G was associated with inferior disease specific survival determined from initiation of IO treatment to cancer specific death. As exemplified in Figure 13 high HLA-G Exon 8 mRNA expression (>= 28.545) had significant worse outcome with 11 HLA-G Exon 8 positive patients having a survival probability of only 25% after 2 years, while the 9 HLA-G Exon 8 negative patients had a survival probability of 100% after 2 years (p=0.0068).

As the examined HLA-G specific Exon 8 region is not translated into protein further confirmatory analysis has been performed by determining the Exon 3 region of HLA-G, which is part of the translated region close to the signal peptide of HLA-G.

As depicted in Figure 14 high HLA-G Exon 3 mRNA expression (>= 26.535) was significantly associated with inferior disease specific survival with 10 HLA-G Exon 3 positive patients having a survival probability of only 15% after 2 years, while 10 HLA-G Exon 3 negative patients had a survival probability of 100% after 2 years (p=0,0013). This resembles the predictive value of HLA-G Exon 8 mRNA expression and further proves that HLA-G expression is associated with worse outcome despite treatment with check point inhibiting IO drugs in advanced and node positive disease situations.

Next it was examined whether other HLA genes, being classical or non-classical or being known genes or yet assigned to be pseudogenes, were predictive for IO outcome in urothelial bladder cancer.

As one example, assays were developed to quantify the mRNA of the "pseudogene" HLA-L at the similar region at the 3'end of the "pseudogene" analogous to the Exon 8 region of HLA-G. As depicted in Figure 15 high HLA-L Exon 7 mRNA expression (>= 29.89) was associated with inferior disease specific survival with 10 HLA-L Exon 7 positive patients having a survival probability of only 30% after 2 years, while 10 HLA-L Exon 7 negative patients had a survival probability of 80% after 2 years. However, this association did not reach statistical significance by log-rank test due to crossing of the survival curves. It can be argued, that on the one hand the sample size is still low, on the other hand the log-rank test might not be valid in this case, as a very early case after 1 month does have an exaggerated effect on the p-value and therefore might not be optimal to assess risk.

This indicates that not only HLA-G, but also other HLA genes and/or pseudogenes are associated with worse outcome despite treatment with check point inhibiting IO drugs. From a therapeutic standpoint this indicates, that not only HLA-G but simultaneously other HLA-genes and/or pseudogenes should be targeted to circumvent or break resistance towards IO drugs.

Next it was examined whether HLA genes are also predictive in most aggressive situations from tumorbiological standpoint, when multiple organs particularly including the lung have already been metastasized as determined by CT scan at diagnosis before IO therapy. As depicted in Figure 16 high HLA-L Exon 7 mRNA expression (>= 30.195) was associated with inferior disease specific survival with 16 HLA-L Exon 7 positive patients having a survival probability of only 0% after 1 year, while 11 HLA-L Exon 7 negative patients had a survival probability of 70% after 1 year (p=0,0418)

In this highly metastasized situation also other "pseudogenes" were significant as exemplified by HLA-H. As depicted in Figure 17 high HLA-H Exon 2/3 mRNA expression (>= 29,95) was associated with inferior disease specific survival with HLA-H Exon 2/3 mRNA positive patients having a survival probability of only 30% after 1 year, while HLA-H Exon 2/3 mRNA negative patients had a survival probability of 80% after 1 year.

## Claims

1. A method for predicting whether a subject having urothelial cancer responds to an anti-PD-1 or anti-PD-L1 immune checkpoint therapy,
wherein the method comprises
(A) determining the level(s) of at least one nucleic acid molecule and/or at least one protein or peptide in a sample obtained from said subject,
wherein the at least one nucleic acid molecule is selected from nucleic acid molecules
(a) encoding a polypeptide comprising or consisting of the amino acid sequence of any one of SEQ ID NOs 2 and 4 to 6,
(b) consisting of the nucleotide sequence of any one of SEQ ID NOs 8 and 10 to 12,
(c) encoding a polypeptide which is at least 85% identical, preferably at least 90% identical, and most preferred at least 95% identical to the amino acid sequence of (a),
(d) consisting of a nucleotide sequence which is at least 95% identical, preferably at least 96% identical, and most preferred at least 98% identical to the nucleotide sequence of (b),
(e) consisting of a nucleotide sequence which is degenerate with respect to the nucleic acid molecule of (d),
(f) consisting of a fragment of the nucleic acid molecule of any one of (a) to (e), said fragment comprising at least 250 nucleotides, preferably at least 300 nucleotides, more preferably at least 450 nucleotides, and most preferably at least 600 nucleotides, and
(g) corresponding to the nucleic acid molecule of any one of (a) to (f), wherein T is replaced by U, and
wherein the at least one protein or peptide is selected from proteins or peptides being encoded by the nucleic acid molecule of any one of (a) to (g); and
(B) comparing the level(s) of (A) with the level(s) of the at least one nucleic acid molecule and/or the at least one protein or peptide in a sample obtained from one or more subjects that responded to the anti-PD-1 or anti-PD-L1 immune checkpoint therapy or a corresponding pre-determined standard,
wherein increased level(s) of (A) as compared to the level(s) or pre-determined standard of (B) indicate(s) that the subject will not respond to the anti-PD-1 or anti-PD-L1 immune checkpoint therapy and substantially the same or decreased level(s) of (A) as compared to the level(s) of (B) indicate(s) that the subject will respond to the anti-PD-1 or anti-PD-L1 immune checkpoint therapy ; or
(B') comparing the level(s) of (A) with the level(s) of the at least one nucleic acid molecule and/or the at least one protein or peptide in a sample obtained from one or more subjects that did not respond to the anti-PD-1 or anti-PD-L1 immune checkpoint therapy or a corresponding pre-determined standard,
wherein decreased level(s) of (A) as compared to the level(s) or pre-determined standard of (B') indicate(s) that the subject will respond to the anti-PD-1 or anti-PD-L1 immune checkpoint therapy and substantially the same or increased level(s) of (A) as compared to the level(s) of (B') indicate(s) that the subject will not respond to the anti-PD-1 or anti-PD-L1 immune checkpoint therapy.

2. The method of claim 1, wherein any one of SEQ ID NOs 2 and 4 to 6 is SEQ ID NO: 5 or 6, and any one of SEQ ID NOs 8 and 10 to 12 is SEQ ID NO: 11 or 12.

3. The method of claim 1 or 2, further comprising determining the mRNA expression level or the protein level of one or more selected from ErbB2, EGFR, CD20, CTLA4, IDO1, LAG3, TIM3, TIM-4, CXCL9, CXCL13, TIGIT, BTLA, CD137, OX40, VISTA, B7-H7, CD27, GITR, TGF-ß Signaling pathway, IL-15, PD-1 and PD-1L, preferably of PD-1 or PD-1L.

4. The method of any one of claims 1 to 3, wherein the immune checkpoint inhibitor is selected from the group consisting of Nivolumab, Pembrolizumab, Cemiplimab, and Atezolizumab,.

5. A method for preparing a kit for predicting whether a subject having urothelial cancer responds to an anti-PD-1 or anti-PD-L1 immune checkpoint therapy,
wherein the method comprises combining
means for the detection of the level(s) of at least one nucleic acid molecule as defined in claim 1 or 2 and/or at least one protein or peptide as defined in claim 1 or 2, and instructions how to use the kit.

6. The method of claim 5, wherein the means comprise primer pairs and optionally a hydrolysis probe used for the specific detection of at least one nucleic acid molecule as defined in claim 1.

## Patentansprüche

1. Verfahren zur Vorhersage, ob ein Individuum mit Urothelkarzinom auf eine Anti-PD-1- oder Anti-PD-L1-Immun-Checkpoint-Therapie anspricht,
wobei das Verfahren umfasst:
(A) die Bestimmung des/der Spiegel(s) mindestens eines Nukleinsäuremoleküls und/oder mindestens eines Proteins oder Peptids in einer von dem Individuum gewonnenen Probe,
wobei das mindestens eine Nukleinsäuremolekül ausgewählt ist aus Nukleinsäuremolekülen, die
(a) für ein Polypeptid kodieren, das die Aminosäuresequenz einer der SEQ ID NOs 2 und 4 bis 6 umfasst oder aus dieser besteht,
(b) aus der Nukleotidsequenz einer der SEQ ID NOs 8 und 10 bis 12 bestehen,
(c) für ein Polypeptid kodieren, das zu mindestens 85 %, vorzugsweise zu mindestens 90 % und am stärksten bevorzugt zu mindestens 95 % identisch mit der Aminosäuresequenz von (a) ist,
(d) aus einer Nukleotidsequenz bestehen, die zu mindestens 95 %, vorzugsweise zu mindestens 96 % und am stärksten bevorzugt zu mindestens 98 % identisch mit der Nukleotidsequenz von (b) ist,
(e) aus einer Nukleotidsequenz bestehen, die in Bezug auf das Nukleinsäuremolekül von (d) degeneriert ist,
(f) aus einem Fragment des Nukleinsäuremoleküls von einem aus (a) bis (e) bestehen, wobei das Fragment mindestens 250 Nukleotide, vorzugsweise mindestens 300 Nukleotide, stärker bevorzugt mindestens 450 Nukleotide und am stärksten bevorzugt mindestens 600 Nukleotide umfasst, und
(g) dem Nukleinsäuremolekül von einem aus (a) bis (f) entsprechen, wobei T durch U ersetzt ist, und
wobei das mindestens eine Protein oder Peptid ausgewählt ist aus Proteinen oder Peptiden, die durch das Nukleinsäuremolekül von einem aus (a) bis (g) kodiert werden; und
(B) Vergleichen des/der Spiegel(s) von (A) mit dem/den Spiegel(n) des mindestens einen Nukleinsäuremoleküls und/oder des mindestens einen Proteins oder Peptids in einer Probe, die von einem oder mehreren Individuen gewonnen wurde, die auf die Anti-PD-1- oder Anti-PD-L1-Immun-Checkpoint-Therapie angesprochen haben, oder mit einem entsprechenden vorab festgelegten Standard,
wobei erhöhte(r) Spiegel von (A) im Vergleich zu dem/den Spiegel(n) oder dem vorab festgelegten Standard von (B) darauf hinweisen, dass das Individuum nicht auf die Anti-PD-1- oder Anti-PD-L1-Immun-Checkpoint-Therapie ansprechen wird, und im Wesentlichen gleiche oder verringerte(r) Spiegel von (A) im Vergleich zu dem/den Spiegel(n) von (B) darauf hinweisen, dass das Individuum auf die Anti-PD-1- oder Anti-PD-L1-Immun-Checkpoint-Therapie ansprechen wird; oder
(B') Vergleichen des/der Spiegel(s) von (A) mit dem/den Spiegel(n) des mindestens einen Nukleinsäuremoleküls und/oder des mindestens einen Proteins oder Peptids in einer Probe, die von einem oder mehreren Individuen gewonnen wurde, die nicht auf die Anti-PD-1- oder Anti-PD-L1-Immun-Checkpoint-Therapie angesprochen haben, oder mit einem entsprechenden vorab festgelegten Standard,
wobei verringerte(r) Spiegel von (A) im Vergleich zu dem/den Spiegel(n) oder dem vorab festgelegten Standard von (B') darauf hinweisen, dass das Individuum auf die Anti-PD-1- oder Anti-PD-L1-Immun-Checkpoint-Therapie ansprechen wird, und im Wesentlichen gleiche oder erhöhte(r) Spiegel von (A) im Vergleich zu dem/den Spiegel(n) von (B') darauf hinweisen, dass das Individuum nicht auf die Anti-PD-1- oder Anti-PD-L1-Immun-Checkpoint-Therapie ansprechen wird.

2. Verfahren nach Anspruch 1, wobei eines der SEQ ID NOs 2 und 4 bis 6 SEQ ID NO: 5 oder 6 ist und eines der SEQ ID NOs 8 und 10 bis 12 SEQ ID NO: 11 oder 12 ist.

3. Verfahren nach Anspruch 1 oder 2, weiterhin umfassend die Bestimmung des mRNA-Expressionsspiegels oder des Proteinspiegels von einem oder mehreren, ausgewählt aus ErbB2, EGFR, CD20, CTLA4, IDO1, LAG3, TIM3, TIM-4, CXCL9, CXCL13, TIGIT, BTLA, CD137, OX40, VISTA, B7-H7, CD27, GITR, TGF-ß-Signalweg, IL-15, PD-1 und PD-1L, vorzugsweise von PD-1 oder PD-1L.

4. Verfahren nach einem der Ansprüche 1 bis 3, wobei der Immun-Checkpoint-Inhibitor ausgewählt ist aus der Gruppe bestehend aus Nivolumab, Pembrolizumab, Cemiplimab und Atezolizumab.

5. Verfahren zur Herstellung eines Kits zur Vorhersage, ob ein Individuum mit Urothelkarzinom auf eine Anti-PD-1- oder Anti-PD-L1-Immun-Checkpoint-Therapie anspricht,
wobei das Verfahren das Zusammenfügen umfasst von
Mitteln zum Nachweis des/der Niveaus von mindestens einem Nukleinsäuremolekül gemäß Anspruch 1 oder 2 und/oder mindestens einem Protein oder Peptid gemäß Anspruch 1 oder 2, sowie Anweisungen zur Verwendung des Kits.

6. Verfahren nach Anspruch 5, wobei die Mittel Primerpaare und optional eine Hydrolysesonde umfassen zur Verwendung für den spezifischen Nachweis von mindestens einem Nukleinsäuremolekül gemäß Anspruch 1.

## Revendications

1. Méthode pour prédire si un sujet ayant un cancer urothélial répond à une thérapie par inhibiteur de point de contrôle immunitaire anti-PD-1 ou anti-PD-L1,
laquelle méthode comprend
(A) la détermination du ou des niveaux d'au moins une molécule d'acide nucléique et/ou d'au moins une protéine ou un peptide dans un échantillon obtenu auprès dudit sujet,
dans laquelle l'au moins une molécule d'acide nucléique est choisie parmi les molécules d'acide nucléique
(a) codant un polypeptide comprenant ou consistant en la séquence d'acides aminés de l'une quelconque des SEQ ID NO : 2 et 4 à 6,
(b) consistant en la séquence d'acides nucléiques de l'une quelconque des SEQ ID NO : 8 et 10 à 12,
(c) codant un polypeptide qui est identique à au moins 85 %, de préférence identique à au moins 90 %, et tout spécialement identique à au moins 95 % à la séquence d'acides aminés de (a),
(d) consistant en une séquence de nucléotides qui est identique à au moins 95 %, de préférence identique à au moins 96 %, et tout spécialement identique à au moins 98 % à la séquence de nucléotides de (b),
(e) consistant en une séquence de nucléotides qui est dégénérée par rapport à la molécule d'acide nucléique de (d),
(f) consistant en un fragment de la molécule d'acide nucléique de l'un quelconque parmi (a) à (e), ledit fragment comprenant au moins 250 nucléotides, de préférence au moins 300 nucléotides, mieux encore au moins 450 nucléotides, et tout spécialement au moins 600 nucléotides, et
(g) correspondant à la molécule d'acide nucléique de l'un quelconque parmi (a) à (f) dans laquelle T est remplacé par U, et
dans laquelle l'au moins une protéine ou un peptide est choisi parmi les protéines ou les peptides qui sont codés par la molécule d'acide nucléique de l'un quelconque parmi (a) à (g) ; et
(B) la comparaison du ou des niveaux de (A) avec le ou les niveaux de l'au moins une molécule d'acide nucléique et/ou de l'au moins une protéine ou un peptide dans un échantillon obtenu auprès d'un ou plusieurs sujets qui ont répondu à la thérapie par inhibiteur de point de contrôle immunitaire anti-PD-1 ou anti-PD-L1 ou un standard prédéterminé correspondant,
dans laquelle un ou plusieurs niveaux accrus de (A) en comparaison avec le ou les niveaux ou le standard prédéterminé de (B) indiquent que le sujet ne va pas répondre à la thérapie par inhibiteur de point de contrôle immunitaire anti-PD-1 ou anti-PD-L1, et un ou plusieurs niveaux pratiquement identiques ou réduits de (A) en comparaison avec le ou les niveaux de (B) indiquent que le sujet va répondre à la thérapie par inhibiteur de point de contrôle immunitaire anti-PD-1 ou anti-PD-L1; ou
(B') la comparaison du ou des niveaux de (A) avec le ou les niveaux de l'au moins une molécule d'acide nucléique et/ou de l'au moins une protéine ou un peptide dans un échantillon obtenu auprès d'un ou plusieurs sujets qui n'ont pas répondu à la thérapie par inhibiteur de point de contrôle immunitaire anti-PD-1 ou anti-PD-L1 ou un standard prédéterminé correspondant,
dans laquelle un ou plusieurs niveaux réduits de (A) en comparaison avec le ou les niveaux ou le standard prédéterminé de (B') indiquent que le sujet va répondre à la thérapie par inhibiteur de point de contrôle immunitaire anti-PD-1 ou anti-PD-L1, et un ou plusieurs niveaux pratiquement identiques ou accrus de (A) en comparaison avec le ou les niveaux de (B') indiquent que le sujet ne va pas répondre à la thérapie par inhibiteur de point de contrôle immunitaire anti-PD-1 ou anti-PD-L1.

2. Méthode selon la revendication 1, dans laquelle l'une quelconque parmi les SEQ ID NO : 2 et 4 à 6 est la SEQ ID NO : 5 ou 6, et l'une quelconque parmi les SEQ ID NO : 8 et 10 à 12 est la SEQ ID NO : 11 ou 12.

3. Méthode selon la revendication 1 ou 2, comprenant en outre la détermination du niveau d'expression d'ARNm ou le niveau de protéine d'un ou plusieurs choisis parmi ErbB2, EGFR, CD20, CTLA4, IDO1, LAG3, TIM3, TIM-4, CXCL9, CXCL13, TIGIT, BTLA, CD137, OX40, VISTA, B7-H7, CD27, GITR, la voie de signalisation de TGF-β, IL-15, PD-1 et PD-1L, de préférence de PD-1 ou de PD-1L.

4. Méthode selon l'une quelconque des revendications 1 à 3, dans laquelle l'inhibiteur de point de contrôle immunitaire est choisi dans le groupe constitué par le nivolumab, le pembrolizumab, le cémiplimab et l'atézolizumab.

5. Méthode pour préparer un kit pour prédire si un sujet ayant un cancer urothélial répond à une thérapie par inhibiteur de point de contrôle immunitaire anti-PD-1 ou anti-PD-L1, laquelle méthode comprend la combinaison
de moyens pour la détection du ou des niveaux d'au moins une molécule d'acide nucléique comme défini dans la revendication 1 ou 2 et/ou d'au moins une protéine ou un peptide comme défini dans la revendication 1 ou 2, et d'instructions sur la manière d'utiliser le kit.

6. Méthode selon la revendication 5, dans laquelle les moyens comprennent des paires d'amorces et éventuellement une sonde d'hydrolyse servant à la détection spécifique d'au moins une molécule d'acide nucléique comme défini dans la revendication 1.
